# EUROPÄISCHE PATENTSCHRIFT

(11) **EP 2 725 026 B1**
(45) Veröffentlichungstag und Bekanntmachung des Hinweises auf die Patenterteilung: **06.09.2017**
(21) Anmeldenummer: 12190324.9
(22) Anmeldetag: 29.10.2012
(51) Int. Cl.: A23L 27/20, A23L 27/00

(54) **HETEROZYKLISCHE NEOFLAVONOIDE MIT GESCHMACKSMASKIERENDEN EIGENSCHAFTEN**
HETEROCYCLIC NEOFLAVONOIDS WITH TASTE MASKING PROPERTIES
NÉOFLAVONOÏDES HÉTÉROCYCLIQUES COMME MASQUEURS DE GOÛT

(43) Veröffentlichungstag der Anmeldung: 30.04.2014
(73) Patentinhaber: Symrise AG, 37603 Holzminden (DE)
(72) Erfinder: Backes, Michael, 37603 Holzminden (DE); Vössing, Tobias, 37688 Beverungen (DE); Ley, Jakob, 37603 Holzminden (DE); Paetz, Susanne, 37671 Höxter (DE)
(74) Vertreter: Fabry, Bernd

(56) Entgegenhaltungen:
- EP-A2- 1 258 200
- WO-A2-2013/036912
- GB-A- 1 486 001
- DATABASE CA [Online] CHEMICAL ABSTRACTS SERVICE, COLUMBUS, OHIO, US; 2012, KIM, SEONG GON: "Method for the preparation of optically active chromane derivative", XP002689086, gefunden im STN Database accession no. 2012:1167380 -& KR 2012 0088031 A (KYONGGI UNIVERSITY, INDUSTRY & ACADEMIC COOPERATION FOUNDATION, S. KOR) 8. August 2012 (2012-08-08)
- PRABHAKAR, MADDELA ET AL: "Michael-type adducts of 3-carbethoxycoumarins via Diels-Alder reaction: tandem ring construction of furopyranochroman-2-one skeletons", SYNLETT, CODEN: SYNLETT; ISSN: 0936-5214, Nr. 6, 2010, Seiten 947-951, XP002689087,
- YU, XIAO-QIANG ET AL: "Rhodium-catalyzed 1,4-addition of lithium 2-furyltriolborates to unsaturated ketones and esters for enantioselective synthesis of .gamma.-oxo-carboxylic acids by oxidation of the furyl ring with ozone", CHEMISTRY--AN ASIAN JOURNAL, CODEN: CAAJBI; ISSN: 1861-4728, Bd. 6, Nr. 3, 2011, Seiten 932-937, XP002689088,
- SHUKLA, MANOJKUMAR R. ET AL: "Synthesis of substituted (.+-.)-3,4- dihydrocoumarins using H-Y", SYNTHETIC COMMUNICATIONS, CODEN: SYNCAV; ISSN: 0039-7911, Bd. 30, Nr. 1, 2000, Seiten 39-42, XP002689089,

## Beschreibung

### Gebiet der Erfindung

Die Erfindung befindet sich auf dem Gebiet der Aromastoffe und betrifft neue Flavonoide mit geschmacksmaskierenden Eigenschaften.

### Stand der Technik

Nahrungs- und Genussmittel enthalten häufig eine Vielzahl verschiedener Bitterstoffe, die zwar einerseits in bestimmten Lebensmitteln in Maßen erwünscht sind und zu deren charakteristischen Geschmack beitragen (z.B. Coffein in Tee oder Kaffee, Chinin in so genannten Bitter-Lemon-Getränken, Bitterstoffe aus Hopfen in Bier), andererseits den Wert aber auch stark mindern können. Dazu gehören z.B. Flavonoidglycoside und Limonoide in Zitrus-Säften, der bittere Nachgeschmack vieler hochintensiver Süßstoffe wie Aspartam, Cyclamat, Acesulfam K, Rebaudiosid A, Glyccyrhizinsäure oder Saccharin sowie der unangenehme Geschmack, den hydrophobe Aminosäuren und Peptide in Käse verursachen können.

Bittergeschmack wird regelmäßig durch einzelne Stoffe verursacht, die an spezielle Bitterrezeptoren auf Geschmackszellen, die in den so genannten Geschmacksknospen auf der Zunge zu finden sind, binden und über neurochemische Kaskaden ein Signal an das Gehirn senden, dass eine Abwehrreaktion und einen negativen Geschmackseindruck verursacht (vgl. Meyerhof, Reviews of Physiology, Biochemistry and Pharmacology 2005, 154, 37-72**).**

Adstringierender Geschmack wird in der Regel durch Fällung von Prolin-reichen Proteinen im Speichel durch Adstringenzien, z.B. Metallsalze, Polyphenole wie (Gallo-)Catechine, Proanthocyanidine, andere Flavonoide oder Tannine, verursacht. Der normalerweise als "Schmiermittel" dienende homogene Speichel enthält dann denaturierte Proteine, die die Gleitfähigkeit herabsetzen und dadurch ein raues oder trockenes, auch als adstringierend empfundenes Gefühl im Mund hinterlassen (vgl. Am. J. Clin. Nutr. 2005, 81, 330S-335S**).**

Zur Senkung des Gehalts an Bitterstoffen in Lebensmitteln, die entweder bereits im Ausgangsmaterial enthalten sind, wie z.B. in Zitrusfrüchten oder während des Herstellungsprozesses entstehen, wie Beispielsweise bei der Käseherstellung, ist daher oft eine nachträgliche Behandlung nötig. Dies kann zum einen extraktiv geschehen, wie bei der Entcoffeinierung von Tee bzw. Kaffee, oder enzymatisch, z.B. Behandlung von Orangensaft mit einer Glycosidase zur Zerstörung des bitteren Naringins oder Spaltung von Gallusestern der Catechine zu den freien Catechinen mit Esterasen oder Einsatz von speziellen Peptidasen bei der Reifung von Käse. Diese Behandlung ist belastend für das Produkt, erzeugt Abfallstoffe und bedingt z.B. auch Lösungsmittelreste und andere Rückstände (Enzyme) in den Produkten.

Daher ist es wünschenswert, Stoffe zu finden, die unangenehme Geschmackseindrücke, insbesondere bittere, adstringierende und/oder metallische Geschmackseindrücke wirkungsvoll verändern, vermindern oder gar unterdrücken können, ohne dabei die Qualität eines entsprechenden Lebensmittels bzw. einer entsprechenden zum Verzehr geeigneten Zubereitung durch zusätzliche Prozessschritte zu beeinflussen.

Auch bei vielen pharmazeutischen Wirkstoffen ist die Unterdrückung des bitteren Geschmacks besonders wichtig. Dadurch kann die Bereitschaft der Patienten, insbesondere bei bitterempfindlichen Patienten wie Kindern, die pharmazeutische Zubereitung oral zu sich zu nehmen, deutlich erhöht werden. Viele pharmazeutische Wirkstoffe, beispielsweise Aspirin, Salicin, Paracetamol, Ambroxol, Antibiotika wie Oxafloxazin oder Chinin, sowie eine ganze Reihe weiterer pharmazeutisch aktiver Verbindungen, weisen einen ausgeprägten bitteren, adstringierenden und/oder metallischen Geschmack und/oder Nachgeschmack auf. Bittere Flavonoidglycoside, vor allem auch das Naringin, werden in Zubereitungen zur Senkung von Blutlipiden sowie als Antioxidantien verwendet.

Des Weiteren weisen auch verschiedene nicht-nutritive, hochintensive Süßstoffe oft geschmackliche Probleme auf. So sind sie zwar durch ihre geringe Einsatzkonzentration gut geeignet, Süße in Nahrungsmittel zu bringen, zeigen jedoch oft geschmackliche Probleme durch im Vergleich zu Zucker unähnliche Zeit-Intensitätsprofile (z.B. Sucralose, Stevioside, Cyclamat), einen bitteren und/oder adstringierenden Nachgeschmack (z.B. Acesulfam K, Saccharin, Steviosid, Rebaudiosid A, Rebaudiosid C) und/oder ausgeprägte zusätzliche Aromaeindrücke (z.B. Glycyrrhizinsäureammoniumsalz). Insbesondere bei süßen, kalorienfreien oder nahezu kalorienfreien Lebensmittel, beispielsweise Getränken, die mit Hilfe solcher Süßstoffe hergestellt wurden, senkt dieser unangenehme Neben- und/oder Nachgeschmack häufig die sensorische Akzeptanz und sollte daher maskiert werden.

Es sind bereits Konzepte zur zumindest teilweisen Bitter-Reduktion bekannt (z.B. in Chemosensory Perception 2008, 1(1): 58-77**).** Dazu gehören beispielsweise die Entfernung des Bitterstoffes aus dem Lebensmittel, wie beispielsweise bei der Entbitterung von Zitrussäften, die Verwendung von Verkapselungssystemen oder das Überdecken von bitter schmeckenden Verbindungen mit Hilfe anderer Geschmacks- oder Aromastoffe, wie z.B. mit Süßstoffen (vgl. Recent Patents on Drug Delivery and Formulation, 2009, 3, 26-39**).** Die beschriebenen Lösungsansätze zeigen aber alle in der Anwendung regelmäßig und zum Teil erhebliche Limitationen, wie teure Rohstoffe, unerwünschte Nebeneffekte (z.B. gleichzeitige Unterdrückung der Süße, gleichzeitig auftretender salziger Geschmack etc.) und/oder Löslichkeitsprobleme, so dass weiterhin Bedarf an natürlichen, einfach zu verwendenden bzw. einfach in oral konsumierbare Zubereitungen einarbeitbare maskierenden Substanzen, insbesondere an Bittermaskierungs-Lösungen, besteht.

Das Dokument EP 1 258 200 A2 betrifft die Verwendung von hydroxysubstituierten 2-Phenyl-chroman-4-onen der allgemeinen Formel (I), 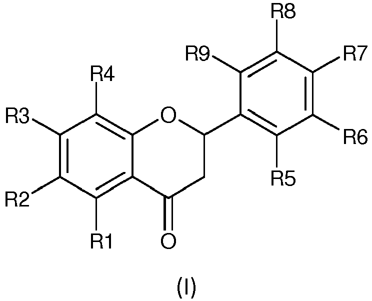 wobei
R¹ bis R⁹ unabhängig voneinander Wasserstoffatome, Hydroxygruppen, Methyl-, Ethyl-, 1-Propyl-, 2-Propyl-, Methoxy-, Ethoxy-, 1-Propyloxy- oder 2-Propyloxygruppen bedeuten können, mit der Maßgabe, dass mindestens einer der Reste R¹ bis R⁹ eine Hydroxygruppe darstellt,
deren Salzen und Stereoisomeren sowie Gemischen derselben zur Maskierung oder Verminderung des bitteren und/oder metallischen Geschmackseindrucks.

Die komplexe Aufgabe der vorliegenden Erfindung hat somit darin bestanden, Substanzen (einzelne Stoffe oder Stoffgemische) zu finden, die eine Veränderung oder Maskierung (d.h. Verminderung oder Unterdrückung) eines bitteren, adstringierenden und/oder metallischen Geschmackseindrucks unangenehm schmeckender Stoffe in zum Verzehr geeigneten Zubereitungen, insbesondere in Nahrungs- und Genussmitteln oder pharmazeutischen Zubereitungen, ermöglichen.

Vorzugsweise sollten die gesuchten Substanzen einen bitter-maskierenden Effekt gegen unterschiedliche Bitterstoffe aufweisen. Dabei sollten die gesuchten Substanzen vorzugsweise den sonstigen, erwünschten Geschmackseindruck einer zum Verzehr geeigneten Zubereitung nicht oder nicht wesentlich beeinflussen. Bevorzugt sollten die gesuchten Substanzen in einer breiten Produktpalette anwendbar und/oder leicht zugänglich sein.

### Beschreibung der Erfindung

In einer ersten Ausführungsform betrifft die Erfindung heterozyklische Neoflavonoide der Formel (II) 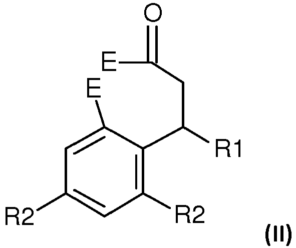 in der
E jeweils OH oder beide E zusammen Sauerstoff bedeuten,
R¹ einen 6-gliedrigen heteroaromatischen Rest darstellt, der ausgewählt ist aus der Gruppe bestehend aus, 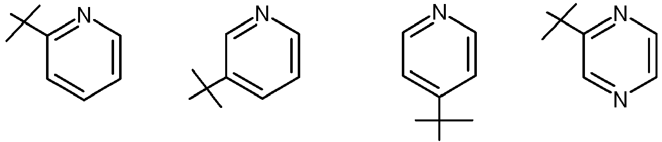
R² jeweils Wasserstoff, Hydroxy, Ethoxy, n-Propoxy oder iso-Propoxy bedeutet, und deren physiologisch verträgliche Salze.

Überraschenderweise wurde gefunden, dass die heterozyklischen Neoflavonoide der Erfindung unangenehme, insbesondere bittere, adstringierende und/oder metallische, Geschmackseindrücke einer Vielzahl von unangenehm schmeckenden Stoffen und oral konsumierbaren Zubereitungen maskieren, d.h. verringern oder sogar vollständig unterdrücken, können. Entsprechendes gilt auch für die nachfolgend beschriebenen erfindungsgemäßen Zubereitungen. Unter Maskieren oder Maskierung wird im Rahmen des vorliegenden Textes eine Reduzierung, d.h. eine Verminderung, bzw. eine vollständige Unterdrückung verstanden. Das Verändern oder Maskieren des unangenehmen Geschmackseindrucks bedeutet damit im Ergebnis regelmäßig eine Geschmacksverbesserung, insbesondere in Bezug auf bittere, adstringierende und/oder metallische Geschmackseindrücke.

Im Rahmen der vorliegenden Erfindung ist es möglich durch den Einsatz der besagten heterozyklischen Neoflavonoide insbesondere den bitteren Geschmackseindruck von Methylxanthinen wie z.B. Coffein, Theobromin, Alkaloiden, wie z.B. Chinin, Flavonoiden wie z.B. Naringin, (Gallo-)Catechinen und deren Gallaten, (Gallo-)-Epicatechinen und deren Gallaten, Phenolen wie z.B. Arbutin, Salicin, oder auch anorganischen Salzen wie Kaliumchlorid oder Magnesiumsulfat zu maskieren, wobei es besonders vorteilhaft ist, dass die erfindungsgemäß zu verwendenden Verbindungen, deren Gemische und/oder deren Salze in den bevorzugt verwendeten geringen Konzentrationen nahezu keinen Eigengeschmack besitzen.

### Heterozyklische Neoflavonoide

Die Konfiguration an dem chiralen Kohlenstoffatom der Verbindungen der Formel (II), 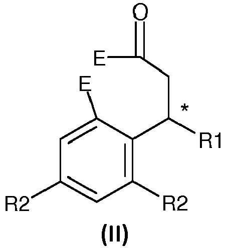 d.h. an der mit einem "*" markierten Position im obigen Formelbild **(II),** kann dabei (R) oder (S) sein. Dies gilt auch für die nachfolgenden Ausführungen und nachfolgend dargestellten Strukturformeln der erfindungsgemäß einzusetzenden Verbindungen. Die Verbindungen der Formel **(II)** können dabei in bevorzugten Ausgestaltungen als reine Enantiomere oder als Mischungen von Enantiomeren in jedem beliebigen Verhältnis zueinander miteinander kombiniert werden. In einer bevorzugten Ausgestaltung werden die Verbindungen der Formel **(II)** in Form von racemischen Gemischen, d.h. als Racemate eingesetzt.

R¹ bedeutet einen 6-gliedrigen heteroaromatischen Rest ausgewählt aus der Gruppe bestehend aus, 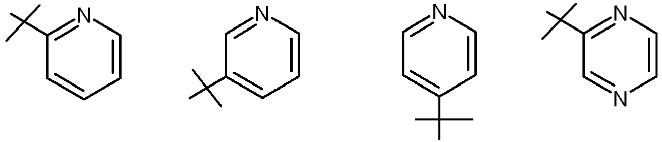 wobei die gestrichelte Linie die Bindung markiert, welche den Rest mit dem in der Formel **(II)** benachbarten C-Atom verknüpft.

R² bedeutet Wasserstoff, Hydroxy, Ethoxy, n-Propoxy, oder iso-Propoxy. In besonders bevorzugter Weise steht R² für Hydroxyl oder eine der folgenden Strukturen. 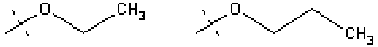 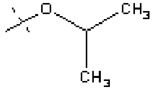 wobei die gestrichelte Linie die Bindung markiert, welche den Rest mit dem in der Formel **(II)** benachbarten C-Atom verknüpft. Die erfindungsgemäß einzusetzenden Verbindungen der Formel **(I)** können je nach Bedeutung von E folgenden Strukturformeln **(II-A)** oder **(II-B)** entsprechen, wobei R1 und R2 jeweils die oben angegebene Bedeutung haben:

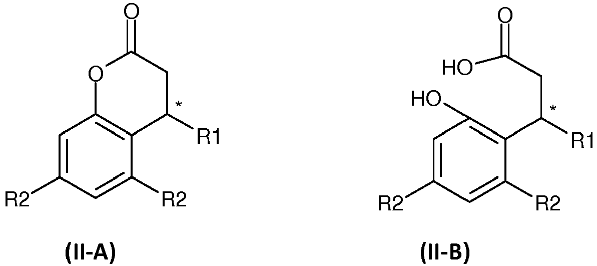

In Abhängigkeit des pH-Wertes kann der Lactonring der Verbindung der Formel **(II-A)** geöffnet werden und die Verbindung der Formel **(II-A)** im Gleichgewicht mit der entsprechenden "offenkettigen" Verbindung der Formel **(II-B)** vorliegen, wie nachfolgend schematisch gezeigt, wobei ein M⁺ (vorzugsweise physiologisch akzeptables) Gegenkation bedeutet (und wobei das Gegenkation bevorzugt die nachfolgend angegebene Bedeutung hat):

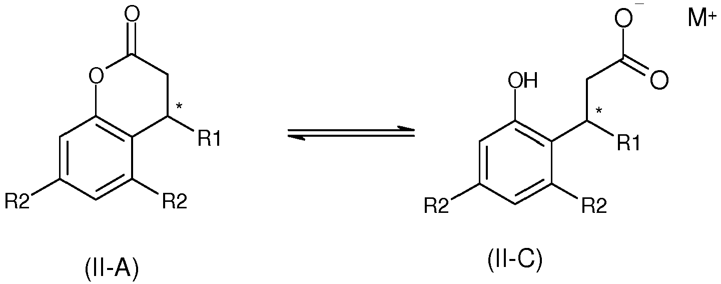

Weiter bevorzugt ist die Verwendung von Verbindungen der Formel **(II-A),** 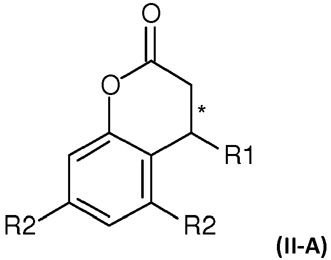 oder eines Salzes einer Verbindung der Formel **(II-A)** oder einer Mischung aus zwei oder mehr unterschiedlichen Verbindungen der Formel **(II-A),** zwei oder mehr unterschiedlichen Salzen von Verbindungen der Formel **(II-A)** oder einer oder mehreren unterschiedlichen Verbindungen der Formel **(II-A)** und einem oder mehreren unterschiedlichen Salzen von einer oder mehreren unterschiedlichen Verbindungen der Formel **(II-A),** wobei
- R¹: einen 6-gliedrigen heteroaromatischen Rest ausgewählt aus der Gruppe bestehend aus, 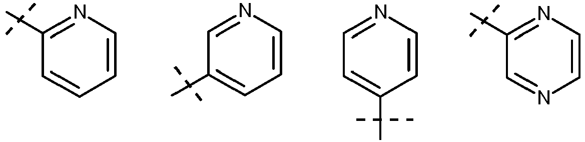 bedeutet, wobei die gestrichelte Linie die Bindung markiert, welche den Rest mit dem in der Formel **(II)** benachbarten C-Atom verknüpft,
- R²: jeweils Wasserstoff, Hydroxy, Ethoxy, n-Propoxy oder iso-Propoxy bedeutet, vorzugsweise H, OH oder OCH₂CH₃.

Dabei gilt als bevorzugte Ausführungsform die Auswahlbedingung, dass mindestens einer der Reste R² verschieden von Wasserstoff sein muss.

Die Konfiguration an dem chiralen Kohlenstoffatom (d.h. an der mit einem "*" markierten Position in den obigen Formelbildern **(II)** und **(II-A))** kann dabei jeweils (R) oder (S) sein.

Bevorzugt weist eine Verbindung der Formel **(II)** bzw. eine Verbindung der Formel **(II-A)** insgesamt eine oder zwei Hydroxygruppen auf, insbesondere bevorzugt insgesamt zwei Hydroxygruppen.

Eine weitere Ausgestaltung der Erfindung ist dabei auf konkrete heterozyklische Neoflavonoide der Formeln (1) bis (6) gerichtet, nämlich
**(1)** (4S)-5,7-Dihydroxy-4-(4-pyridyl)chroman-2-on
**(2)** (4R)-5,7-Dihydroxy-4-(4-pyridyl)chroman-2-on
**(3)** (4S)-5,7-Dihydroxy-4-(3-pyridyl)chroman-2-on
**(4)** (4R)-5,7-Dihydroxy-4-(3-pyridyl)chroman-2-on
**(5)** (4S)-5,7-Dihydroxy-4-(2-pyridyl)chroman-2-on
**(6)** (4R)-5,7-Dihydroxy-4-(2-pyridyl)chroman-2-on 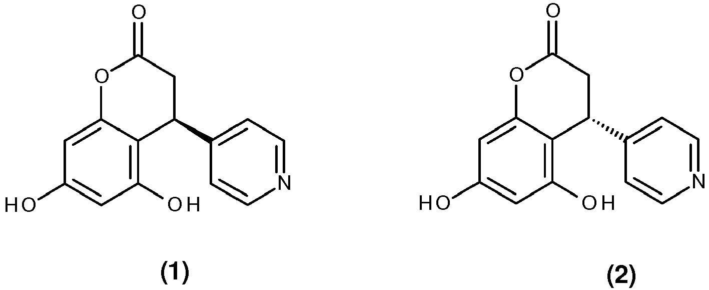 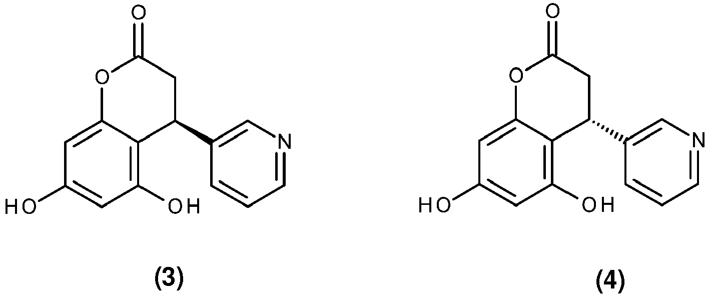 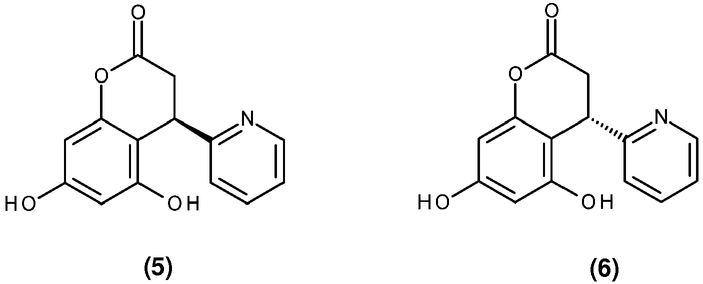
sowie deren physiologisch verträglichen Salze.

Im Kontext der vorliegenden Erfindung sind mit den physiologisch verträglichen Salzen solche gemeint, bei denen deren Kationen ausgewählt sind aus der Gruppe, die gebildet wird von den einfach positiv geladenen Kationen der ersten Haupt- und Nebengruppe, Ammonium und Trialkylammonium, den zweiwertig geladenen Kationen der zweiten Nebengruppe, sowie den dreiwertigen Kationen der 3. Haupt- und Nebengruppe. Bevorzugt sind Gegenkationen ausgewählt aus der Gruppe bestehend aus Na⁺, K⁺, NH₄⁺, Ca²⁺, Mg²⁺, Al³⁺ und Zn²⁺ sowie deren Gemische.

Für den Fall, dass im Einzelfall ein Widerspruch zwischen der vorstehend angegebenen chemischen Nomenklatur und der jeweils dargestellten Strukturformel der erfindungsgemäß bevorzugt einzusetzenden Verbindungen der Formeln **(1)** bis **(6)** bestehen sollte, gilt die Strukturformel.

Die heterozykliscchen Neoflavonoide der Formel (II) sind neu, lassen sich aber grundsätzlich nach den einschlägigen Methoden der organischen Chemie erhalten. Besonders bevorzugt ist die säurekatalysierte Umsetzung (klassisch z.B. unter Verwendung von Schwefelsäure oder Salzsäure) von Phenolderivaten der nachfolgenden Formel **(A)** mit Zimtsäurederivaten der nachfolgenden Formel **(B)** oder der entsprechenden Ester der nachfolgenden Formel **(C)** zu den Verbindungen der Formel **(II),** wie in dem nachfolgenden Schema skizziert: 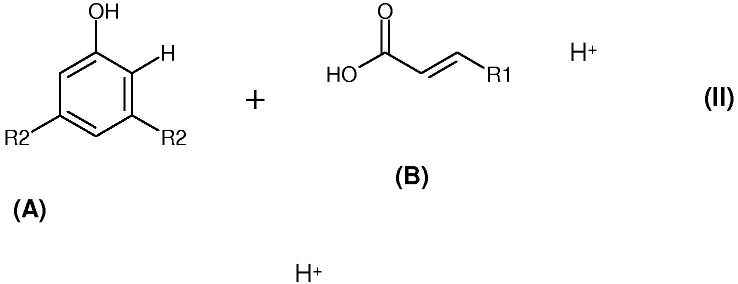 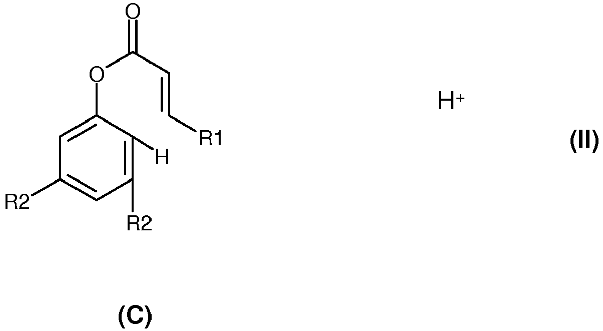 wobei jeweils die Reste R1 und R2 die oben bezüglich der Formel **(II)** angegebene Bedeutung haben.

Des Weiteren können die erfindungsgemäß einzusetzenden heterocyclischen Neoflavanoide auch analog zu der in Synth. Commun. 1987, 17(6) 723-727 beschriebenen Methode hergestellt werden, wie nachfolgend anhand der Reaktion von Meldrumsäure **(E)** mit Phloroglycinol **(D)** unter Zugabe eines Aldehyds (z.B. Pyridin-2-carbaldehyd **(F))** in Gegenwart von Pyridin zur Herstellung eines racemischen Gemisches der erfindungsgemäß einzusetzenden Verbindungen **(5)** und **(6)** schematisch dargestellt.

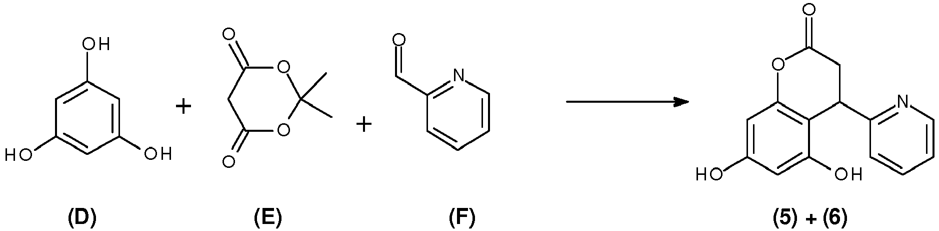

### Gewerbliche Anwendbarkeit

### Zubereitungen zur oralen Aufnahme

Ein weiterer Gegenstand der vorliegenden Erfindung ist auf Zubereitungen zur oralen Aufnahme einschließlich entsprechender Halbfertigprodukte gerichtet, enthaltend
(a) mindestens eines der vorstehend definierten heterozyklischen Neoflavonoide und
(b) mindestens einen Stoff mit bitterem, adstringierendem und/oder metallischem Geschmackseindruck.

### Stoffe mit unangenehmen Geschmackseindrücken

Unangenehm schmeckende Stoffe im Rahmen dieses Textes sind:
(b1) Stoffe, die bitter, adstringierend, pappig, kalkig, staubig, trocken, mehlig, ranzig und/oder metallisch schmecken sowie
(b2) Stoffe, die einen bitteren, adstringierenden, pappigen, kalkigen, staubigen, trockenen, mehligen, ranzigen und/oder metallischen Nachgeschmack aufweisen.

Die vorgenannten unangenehm schmeckenden Stoffe können weitere, nicht unangenehme Geschmacks- und/oder Geruchsqualitäten besitzen. Im Rahmen dieses Textes sind als nicht unangenehme Geschmacksqualitäten bevorzugt die Eindrücke würzig, umami, süß, salzig, sauer, scharf, kühlend, wärmend, brennend oder kribbelnd zu nennen.

Zubereitungen, die Gegenstand der Erfindung sind, enthalten vorzugsweise einen, zwei oder mehrere der folgenden Bitterstoffe:
- Catechine und Proanthocyanidine in einer Gesamtmenge von zumindest 0,01 Gew.-%, vorzugsweise von zumindest 0,05 Gew.-%, bevorzugt im Bereich von 0,075 Gew.-% bis 1 Gew.-%,
- Coffein und Theobromin in einer Gesamtmenge von zumindest 0,005 Gew.-%, vorzugsweise von zumindest 0,01 Gew.-%, bevorzugt von zumindest 0,02 Gew.-%, weiter bevorzugt im Bereich von 0,025 Gew.-% bis 1 Gew.-%,
- Naringin in einer Konzentration von zumindest 0,005 Gew.-%, vorzugsweise von zumindest 0,01 Gew.-%, bevorzugt im Bereich von 0,02 Gew.-% bis 0,5 Gew.-%,
- Süßstoffe in einer Gesamtmenge von zumindest 0,005 Gew.-%, vorzugsweise von zumindest 0,05 Gew.-%, bevorzugt im Bereich von 0,1 Gew.-% bis 2 Gew.-%,
jeweils bezogen auf das Gesamtgewicht der Zubereitung.

Stoffe, die bitter, adstringierend, pappig, kalkig, staubig, trocken, mehlig, ranzig und/oder metallisch schmecken, sind beispielsweise: Xanthinalkaloide, Xanthine (Coffein, Theobromin, Theophyllin), Alkaloide (Chinin, Brucin, Strychnin, Nicotin), phenolische Glycoside (z.B. Salicin, Arbutin), Flavanoidglycoside (z.B. Neohesperidin, Eriocitron, Neoeriocitrin, Narirutin, Hesperidin, Naringin), Chalcone oder Chalconglycoside, Dihydrochalconglycoside (Phloridzin, Trilobatin), hydrolisierbare Tannine (Gallus- oder Elagsäureester von Kohlenhydraten, z.B. Pentagalloylglucose), nichthydrolisierbare Tannine (ggfs. galloylierte Catechine oder Epicatechine und deren Oligomeren, z.B. Proanthyocyanidine oder Procyanidine, Thearubigenin), Flavone und deren Glycoside (z.B. Quercetin, Quercitrin, Rutin, Taxifolin, Myricetin, Myrictrin), andere Polyphenole (γ-Oryzanol, Kaffeesäure oder deren Ester), terpenoide Bitterstoffe (z.B. Limonoide wie Limonin oder Nomilin aus Zitrusfrüchten, Lupolone und Humolone aus Hopfen, Iridoide, Secoiridoide), Absinthin aus Wermut, Amarogentin aus Enzian, metallische Salze (Kaliumchlorid, Natriumsulfat, Magnesiumsalze, Eisensalze, Aluminiumsalze, Zinksalze), pharmazeutische Wirkstoffe (z.B. Fluorchinolon-Antibiotika, Paracetamol, Aspirin, β-Lactam-Antibiotika, Ambroxol, Propylthiouracil [PROP], Guaifenesin), Vitamine (beispielsweise Vitamin H, Vitamine aus der B-Reihe wie Vitamin B1, B2, B6, B12, Niacin, Panthotensäure), Denatoniumbenzoat oder andere Denatoniumsalze, Sucraloseoctaacetat, Harnstoff, ungesättigte Fettsäuren, insbesondere ungesättigte Fettsäuren in Emulsionen, Aminosäuren (z.B. Leucin, Isoleucin, Valin, Tryptophan, Prolin, Histidin, Tyrosin, Lysin oder Phenylalanin), Peptide (insbesondere Peptide mit einer Aminosäure aus der Gruppe Leucin, Isoleucin, Valin, Tryptophan, Prolin oder Phenylalanin am N- oder C-Terminus). Die vorgenannten Stoffe können entweder einzeln oder als Gemisch vorkommen, bevorzugt auch als natürliche Extrakte aus frischen, getrockneten, gerösteten, und/oder fermentierten Pflanzen oder Pflanzenteilen, so z.B. als Extrakte aus Blättern, Früchten, Ästen, Wurzeln, Fruchtschalen, Kernen, Samen z.B. aus Camellia sinensis, Camellia japonica, Coffea ssp., Cocoa theobroma, Vitis vinifera, Citrus ssp. und Hybriden, Poncirus ssp. und Hybriden, Perilla, Humulus lupulus, oder verwandten Arten stammen.

Erfindungsgemäß zu maskierende Bitterstoffe sind dabei insbesondere Xanthine (insbesondere Coffein, Theobromin, Theophyllin), phenolische Glycoside (insbesondere Salicin, Arbutin), Flavanoidglycoside (insbesondere Neohesperedin, Eriocitrin, Neoeriocitrin, Narirutin, Hesperidin, Naringin), Chalcone oder Chalconglycoside, Dihydrochalconglycoside (insbesondere Phloridzin, Trilobatin), hydrolisierbare Tannine (insbesondere Gallus- oder Ellagsäureester von Kohlenhydraten, z.B. Pentagalloylglucose), nichthydrolisierbare Tannine (insbesondere galloylierte Catechine oder Epicatechine und deren Oligomeren, z.B. Proanthyocyanidine oder Procyanidine, Thearubigenin), Flavone und deren Glycoside (insbesondere Quercetin, Quercitrin, Rutin, Taxifolin, Myricetin, Myrictrin), Kaffeesäure oder deren Ester, terpenoide Bitterstoffe (insbesondere Limonin, Nomilin, Lupolone und Humolone), metallische Salze (Kaliumchlorid, Natriumsulfat, Magnesiumsalze, Eisensalze, Aluminiumsalze, Zinksalze), pharmazeutische Wirkstoffe (z.B. Fluorchinolon-Antibiotika, Paracetamol, Aspirin, β-Lactam-Antibiotika, Ambroxol, Propylthiouracil [PROP], Guaifenesin), Aminosäuren (z.B. Leucin, Isoleucin, Valin, Tryptophan, Prolin, Histidin, Tyrosin, Lysin oder Phenylalanin), Peptide (insbesondere Peptide mit einer Aminosäure aus der Gruppe Leucin, Isoleucin, Valin, Tryptophan, Prolin oder Phenylalanin am N- oder C-Terminus).

Speziell bevorzugte zu maskierende Bitterstoffe sind ausgewählt aus der Gruppe bestehend aus Coffein, Theobromin, Chinin, Salicin, Arbutin, Neohesperedin, Eriocitrin, Neoeriocitrin, Narirutin, Hesperidin, Naringin, Phloridzin, Catechin, Epicatechin, Epigallocatechingallat (EGCG), Gallocatechin, Gallocatechin-3-gallat, Procyanidin B2, Procyanidin B5, Procyanidin C1, Thearubigenin, Rutin, Taxifolin, Myricetin, Myrictrin, Kaffeesäure oder deren Ester, Limonin und Nomilin, Aminosäuren (z.B. Leucin, Isoleucin, Valin, Tryptophan, Prolin, Histidin, Tyrosin, Lysin oder Phenylalanin), Peptide mit einer Aminosäure aus der Gruppe Leucin, Isoleucin, Valin, Tryptophan, Prolin oder Phenylalanin am N- oder C-Terminus, Kaliumchlorid, Paracetamol, Aspirin und β-Lactam-Antibiotika.

Stoffe, die einen bitteren, adstringierenden, pappigen, kalkigen, staubigen, trockenen, mehligen, ranzigen und/oder metallischen Neben- und/oder Nachgeschmack haben, können Aroma- oder Geschmackstoffe mit einem nicht unangenehmen Primärgeschmack (beispielsweise süß, salzig, würzig, sauer) und/oder -geruch sein, und z.B. zur Gruppe der Süßstoffe, Zuckeraustauschstoffe oder der Aromastoffe gehören. Beispielsweise seien genannt: Aspartam, Neotam, Superaspartam, Alitam, Saccharin, Sucralose, Tagatose, Monellin, Monatin, Stevioside, Rubusosid, Steviosid, Rebaudiosid A, Rebaudioside C, Thaumatin, Miraculin, Glycyrrhizin (Glycyrrhizinsäure), Glycyrrhetinsäure oder deren Derivate, Cyclamat oder die physiologisch akzeptablen Salze der vorgenannten Verbindungen

Da sich die Bitterintensität verschiedener Bitterstoffe deutlich unterscheidet, wird die Bitterkeit einer Verbindung im Folgenden manchmal in relativen Bitterequivalenten (RBE) angegeben. Als Bezugssubstanz wird hier der bekannte Bitterstoff Coffein verwendet. Die Bestimmung des RBE-Wertes als Maß für die relative Bitterkeit einer Probe erfolgt mit Hilfe einer Skala von 1 bis 10. Eine relative Bitterkeit von 1, d.h. eine RBE-Wert von 1, entspricht dabei der Bitterkeit einer Menge von Coffein in der Dosierung von 100 mg/kg zu untersuchender Probe. Eine relative Bitterkeit von 5, d.h. eine RBE-Wert von 5, entspricht dabei der Bitterkeit einer Menge von Coffein in der Dosierung von 500 mg/kg zu untersuchender Probe. Die zu untersuchende Probe kann dabei in ihrer Zusammensetzung stark variieren. So kann es sich bei der zu untersuchenden Probe beispielsweise um eine der Ernährung, der Mundpflege, dem Genuss dienende Zubereitung, eine orale pharmazeutischen Zubereitung oder eine kosmetischen Zubereitung, beispielsweise um ein Lebensmittel, ein Getränk, ein Kaugummi, ein Mundwasser, ein Bonbon, einen Hustensaft oder eine Tablette handeln.Die verwendete Skala zur Bestimmung der RBE-Werte entspricht ISO 4121 [Sensory Analysis - Guidelines for the use of quantitative response scales; A.3 Beispiel 2].Die Auswahl der Panelisten zur Bestimmung der RBE-Werte erfolgt gemäß ISO 8586-1 [Sensory analysis - General guidance for the selection, training, and monitoring,of assessors - Part 1: Selected assessors]. Die Zahl der Panelisten entspricht dabei ISO 8586-I, 4.2.3 [Number of persons to be selected, zusammen mit ISO 6658 Sensory analysis - Methodology - General guidance - 5.3.5 Scoring (5 oder mehr ausgewählte Panelisten)]. Dabei besteht der entscheidende Vorteil der erfindungsgemäß einzusetzenden heterozyklischen Neoflavonoide, dass auch solche Bitterstoffe in ihrem unangenehmen Geschmack kompensiert werden können, wenn sie in einer Konzentration vorliegen, die mindestens 2 relativen Bitterequivalenten entspricht,

### Konfektioniert Produkte

Unter oralen Zubereitungen, die Gegenstand der vorliegenden Erfindung sind, werden im Folgenden nicht nur solche Produkte verstanden, die der menschlichen Ernährung dienen, sondern auch solche Mittel, die mit der Mundschleimhaut in Kontakt kommen. Somit umfasst diese Bezeichnung Nahrungsmittel auf der einen und Mund- und Zahnreinigungs- und -pflegemittel sowie oral verabreichte pharmazeutische Mittel auf der anderen Seite.

Vorzugsweise handelt es sich bei den oralen Zubereitung um Backwaren, beispielsweise Brot, Trockenkekse, Kuchen, sonstiges Gebäck, Süßwaren (beispielsweise Schokoladen, Schokoladenriegelprodukte, sonstige Riegelprodukte, Fruchtgummi, Hart- und Weichkaramellen, Kaugummi), alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (carbonisierte) fruchthaltige Limonaden, (carbonisierte) isotonische Getränke, (carbonisierte) Erfrischungsgetränke, Nektare, Schorlen, Obst- und Gemüsesäfte, Frucht- oder Gemüsesaftzubereitungen, Instantgetränke (beispielsweise Instant-Kakao-Getränke, Instant-Tee-Getränke, Instant-Kaffeegetränke, Instant-Fruchtgetränke), Fleischprodukte (beispielsweise Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (beispielsweise Frühstückscerealien, Müsliriegel, vorgegarte Fertigreis-Produkte), Milchprodukte (beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolisierte Milchprotein-haltige Produkte), Produkte aus Sojaprotein oder anderen Sojabohnen-Fraktionen (beispielsweise Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Produkte aus anderen pflanzlichen Proteinquellen, beispielsweise Haferprotein-Getränke, Fruchtzubereitungen (beispielsweise Konfitüren, Fruchteis, Fruchtsoßen, Fruchtfüllungen), Gemüsezubereitungen (beispielsweise Ketchup, Soßen, Trockengemüse, Tiefkühlgemüse, vorgegarte Gemüse, eingekochte Gemüse), Knabberartikel (beispielsweise gebackene oder frittierte Kartoffelchips oder Kartoffelteigprodukte, Extrudate auf Mais- oder Erdnussbasis), Produkte auf Fett- und Ölbasis oder Emulsionen derselben (beispielsweise Mayonnaise, Remoulade, Dressings), sonstige Fertiggerichte und Suppen (beispielsweise Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

Alternativ kommen Zahnpasten, Mundwässer, Erkältungsmittel oder Wirkstoffkapseln in Betracht.

Die oral konsumierbaren Produkte im Sinne der Erfindung können auch als Halbfertigware zur Herstellung weiterer oral konsumierbarer Produkte dienen. Die oral konsumierbaren süß schmeckenden Produkte im Sinne der Erfindung können auch in Form von Kapseln, Tabletten (nichtüberzogene sowie überzogene Tabletten, beispielsweise magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Die in den oralen Zubereitungen eingesetzte Gesamtmenge der Komponenten (a) und (b) kann bezogen auf die konfektionierten Endprodukte etwa 0,001 bis etwa 1 und vorzugsweise etwa 0,01 bis 0,5 Gew.-% betragen. Insbesondere kann die Menge der heterozyklischen Neoflavonoide bezogen auf die Halbfertigwaren oder die konfektionierten Endprodukte im Bereich von 0,1 mg/kg (entsprechend 0,1 ppm) bis 1 g/kg vorzugsweise im Bereich von 0,5 bis 900 mg/kg (entsprechend 0,5 bis 900 ppm), bevorzugt im Bereich von 1 bis 500 mg/kg, weiter bevorzugt im Bereich von 3 bis 300 mg/kg, besonders bevorzugt im Bereich von 5 bis 200 mg/kg, am meisten bevorzugt im Bereich von 10 bis 100 mg/kg liegen.

### A. Aromastoffe, Geschmacksstoffe und Geschmackskorrigenzien

Bevorzugt enthalten erfindungsgemäße Zubereitungen, insbesondere erfindungsgemäße zum, vorzugsweise direkten, Verzehr geeignete Zubereitungen, ein oder mehrere weitere Aromastoffe, Geschmackstoffe und/oder Geschmackskorrigenzien, um den Geschmack und/oder Geruch der Zubereitung abzurunden, zu verbessern bzw. zu verfeinern. Geeignete weitere Aromastoffe, Geschmackstoffe und/oder Geschmackskorrigenzien sind dabei vorzugsweise synthetische oder natürliche Aromastoffe, Geschmackstoffe und/oder Geschmackskorrigenzien, dabei vorzugsweise speichelfördernde, prickelnd, scharf und/oder warm schmeckende Substanzen, ätherische Öle oder Pflanzenextrakte, sowie gegebenenfalls zusätzlich zum Verzehr geeignete Hilfs- und Trägerstoffe. Als besonders vorteilhaft ist dabei, dass der bittere, adstringierende und/oder metallische Geschmackseindruck von weiteren Aromastoffen, Geschmackstoffen und/oder Geschmackskorrigenzien zusätzlich verändert und/oder vermindert werden kann und damit das gesamte Aroma- oder Geschmacksprofil einer erfindungsgemäße Zubereitung verbessert wird.

Erfindungsgemäße Zubereitungen enthalten vorzugsweise zumindest einen, zwei, drei, vier, fünf, sechs, sieben, acht, neun, zehn oder mehr weitere Aromastoffe.

Im Rahmen der vorliegenden Erfindung bevorzugt einzusetzende (einen oder mehrere) Aromastoffe werden vorzugsweise gewählt aus der Gruppe bestehend aus: Acetophenon, Allylcapronat, alpha-Ionon, beta-Ionon, Anisaldehyd, Anisylacetat, Anisylformiat, Benzaldehyd, Benzothiazol, Benzylacetat, Benzylalkohol, Benzylbenzoat, beta-Ionon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion^{®}), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

### B. Süßstoffe

Die erfindungsgemäßen oralen Zubereitungen können insbesondere auch Süßstoffe enthalten, beispielsweise seien genannt: Aspartam, Neotam, Superaspartam, Alitam, Saccharin, Sucralose, Tagatose, Monellin, Monatin, Stevioside, Rubusoside, Stevioside, Rebaudiosid A, Rebaudiosid C, Thaumatin, Miraculin, Glycyrrhizin (Glycyrrhizinsäure), Glycyrrhetinsäure oder deren Derivate, Cyclamat oder die physiologisch akzeptablen Salze der vorgenannten Verbindungen.

### C. Getränke

Als orale Zubereitungen, die die erfindungsgemäßen heterozyklischen Neoflavonoide enthalten können, sind insbesondere Getränke zu nennen, die auch karbonisiert sein können. Bevorzugte erfindungsgemäße Getränke sind gegebenenfalls karbonisierte, säure- und fruchthaltige Limonaden (beispielsweise Orangen-, Limonen- oder Zitronentyp), karbonisierte isotonische Getränke (beispielsweise Orangen-, Limonen oder Zitronentyp), karbonisierte, saure Erfrischungsgetränke (beispielsweise Cola-, Zitrone-, Orange-, Limone-, Kirsch-, Apfel-, Vanille-Typ oder deren Mischung), karbonisierte Schorlen, karbonisierte Obst- und Gemüsesäfte, karbonisierte Frucht- oder Gemüsesaftzubereitungen. Dabei bedeutet karbonisiert im Sinne der Erfindung, dass das Getränk natürlich (z.B. aus Gärungsprozessen wie bei der Bierherstellung oder durch Wasser aus Kohlendioxid-haltigen Mineralquellen) eingetragenes Kohlendioxid enthält oder diesem Kohlendioxid während des Herstell- und/oder Abfüllprozesses zugesetzt wurde.

Diese können als bevorzugte Hilfs- oder Trägerstoffe Maltodextrin, Stärke, natürliche oder künstliche Polysaccharide und/oder Pflanzengummen wie modifizierte Stärken oder Gummi Arabicum, für die Aromamischungen zugelassene Lösungsmittel wie beispielsweise Ethanol, 1,2-Propylenglycol, Wasser, Glycerin, Triacetin, Pflanzenöltriglyceride, färbende Mittel, beispielsweise zugelassene Lebensmittelfarbstoffe, färbende Pflanzenextrakte, Stabilisitoren, Konservierungsstoffe, Antioxidantien und viskositäte-beeinflussende Stoffe enthalten.

Besonders bevorzugte oral konsumierbare süß schmeckende Produkte im Sinne der Erfindung sind alkoholische Getränke wie Biermixgetränke, Weinmixgetränke oder sonstige, maximal 5 Vol.-% Alkohol enthaltende Mischgetränke und/oder nichtalkoholische Getränke wie Tee, Eistee (gesüßt, beispielsweise auch mit Kräuteraromen oder Fruchtaromen vom Typ Zitrone, Orange), (carbonisierte) fruchthaltige Limonaden (beispielsweise Orangen-, Limonen- oder Zitronentyp), (carbonisierte) isotonische Getränke (beispielsweise Orangen-, Limonen oder Zitronentyp), (carbonisierte) Erfrischungsgetränke (beispielsweise Cola-, Zitrone-, Orange-, Limone-, Kirsch-, Apfel-, Vanille-Typ oder deren Mischung), Nektare, Schorlen, Milchgetränke, Buttermilchgetränke, Joghurt, Kefir, Molkegetränke, Sojamilch und daraus gefertigte Produkte, Fruchtgetränke mit Sojaprotein, Haferprotein-Getränke, und Instantgetränke (beispielsweise Instant-Kakao-Getränke, Instant-Tee-Getränke, Instant-Kaffee-getränke, Instant-Fruchtgetränke), sogenannte aromatisierte Wässer ("Near Water"-Getränke, wobei letztere eine Süßung haben müssen.

"Near Water"-Getränke im Sinne dieses Textes sind (karbonisierte) Getränke auf (Mineral-)Wasserbasis, die meist klar sind, nur schwach gefärbt werden, oft nur schwach gesüßt sind (weniger als 5 % Sucrose oder Süßstoffe mit einer Süßkraft von weniger als 5 % Sucrose), meist gar nicht oder nur schwach angesäuert sind und dabei einen pH-Bereich von ca. 4 bis 8 umfassen meist nur aromatisiert sind und dabei noch mit Mineralien, Vitaminen und/oder Pflanzenextrakten versehen werden können. Im Gegensatz zu den meisten andere Getränken (z.B. Limonaden, Fruchtsaftgetränken, [Eis-]Teegetränken u.s.w.) steht dabei der "Wasser"-Charakter des Getränks immer noch im Vordergrund.

Bevorzugt sind dabei die Getränke, die einen pH-Wert kleiner 7, besonders bevorzugt kleiner 5, insbesondere bevorzugt kleiner 4 haben.

### D. Mund- und Zahnpflegemittel

Erfindungsgemäße oral konsumierbare süß schmeckende Produkte können auch der Mund- und Zahnreinigung und-pflege dienen. Beispiele hierfür sind Zahnpasten, Zahngele, Zahnpulver, Mundwässer und dergleichen. Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete **Putzkörper** für die erfindungsgemäßen Zahnpasten sind vor allem feinteilige Xerogelkieselsäuren, Hydrogelkieselsäuren, Fällungskieselsäuren, Aluminiumoxidtrihydrat und feinteiliges alpha -Aluminiumoxid oder Mischungen dieser Putzkörper in Mengen von 15 bis 40 Gew.-% der Zahnpasta.

Als **Feuchthaltemittel** kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummiarabicum, Xantham-Gum und Carboxyvinylpolymere (z. B. Carbopol^{®}-Typen) geeignet.

Zusätzlich können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419, DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam^{®}, (L-Aspartyl- L-phenylalanin-methylester), Stiviaextrakte oder deren süßenden Bestandteile, insbesondere Ribeaudioside;
- geschmacksmodulierende Stoffe (beispielsweise Inositolphosphat, Nucleotide wie Guanosinmonophosphat, Adenosinmonophosphat oder andere Stoffe wie Natriumglutamat oder 2-Phenoxypropionsäure),
- Kühlwirkstoffen wie beispielsweise Menthol, Mentholderivate (beispielsweise L-Menthol, L-Menthyllactat, L-Menthylalkylcarbonate, Menthonketale, Menthancarbonsäureamide), 2,2,2-Trialkylessigsäureamiden (beispielsweise 2,2-Diisopropylpropion-säuremethylamid), Icilin-Derivate,
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- wundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

Zur Verbesserung des Fließverhaltens können ferner **Hydrotrope,** wie beispielsweise Ethanol, Isopropylalkohol, oder Polyole eingesetzt werden; diese Stoffe entsprechen weitgehend den eingangs geschilderten Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die unter der Bezeichnung Surfacine^{®} bekannten Silberkomplexe und die in Anlage 6, Teil A und B der Kosmetikverordnung aufgeführten weiteren Stoffklassen.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Aromen** kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### E. Kaugummis

Bei den bevorzugten oralen Zubereitungen kann es sich auch um Kaugummis handeln. Diese Produkte enthalten typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500, auf deren Inhalt hiermit ausdrücklich Bezug genommen wird.

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

### F. Pharmazeutische Zubereitungen

Sofern die oralen Zubereitungen pharmazeutische Mittel darstellen, umfassen diese einen pharmazeutischen Wirkstoff. Vorteilhafte pharmazeutische Wirkstoffe sind beispielsweise steroidale entzündungshemmende Substanzen vom Kortikosteroiden-Typ wie beispielsweise Hydrocortison, Hydrocortison-Derivate wie Hydrocortison-17-butyrat, Dexamethason, Dexamethasonphosphat, Methylprednisolon oder Cortison.

Vorteilhafte nichtsteroidale pharmazeutische Wirkstoffe sind beispielsweise Entzündungshemmer wie Oxicame wie Piroxicam oder Tenoxicam; Salicylate wie Aspirin^{®} (Acetylsalicylsäure), Disalcid, Solprin oder Fendosal; Essigsäure-Derivate wie Diclofenac, Fenclofenac, Indomethacin, Sulindac, Tolmetin, oder Clindanac; Fenamate wie Mefenamic, Meclofenamic, Flufenamic oder Niflumic; Propionsäure-Derivate wie Ibuprofen, Naproxen, Flurbiprofen, Benoxaprofen oder Pyrazole wie Phenylbutazon, Oxyphenylbutazon, Febrazon oder Azapropazon.

Besonders bevorzugte pharmazeutische Zubereitungen sind nicht verschreibungspflichtige Produkte und frei verkäufliche Arzneimittel, sogenannte OTC ("over the counter") - Präparate, enthaltend Wirkstoffe wie Paracetamol, Acetylsalicylsäure oder Ibuprofen, Vitamine (beispielsweise Vitamin H, Vitamine aus der B-Reihe wie Vitamin B1, B2, B6, B12, Niacin, Panthotensäure, vorzugsweise in Form von (Brause)Tabletten oder Kapseln), Mineralien (vorzugsweise in Form von (Brause)Tabletten oder Kapseln) wie Eisensalze, Zinksalze, Selensalze, Produkte enthaltend Wirkstoffe oder Extrakte von Spitzwegerich (beispielsweise in Hustensirup) oder Johanniskraut.

### G. Kapseln

Die oralen Zubereitungen können auch in Emulsionen, in Liposomen, beispielsweise ausgehend von Phosphatidylcholin, in Microsphären, in Nanosphären und insbesondere auch in Kapseln, Granulaten oder Extrudaten aus einer für Lebens- und Genussmittel geeigneten Matrix, beispielsweise aus Stärke, Stärkederivaten, Cellulose oder Cellulosederivaten (beispielsweise Hydroxypropylcellulose) eingearbeitet werden.

In eine weiteren bevorzugten Ausführungsform werden die erfindungsgemäßen Aromamischungen mit einem oder mehreren geeigneten Komplexbildnern, beispielsweise mit Cycloglycanen, beispielsweise Cyclofructanen, Cyclodextrinen oder Cyclodextrinderivaten, bevorzugt α-, γ- und ▪-Cyclodextrin, komplexiert und in dieser komplexierten Form als konfektioniertes Endprodukt, d.h. als orale Zubereitung eingesetzt. Besonders bevorzugt ist eine erfindungsgemäßes oral konsumierbares süß schmeckendes Produkt, bei der die Matrix so gewählt wird, dass die erfindungsgemäße Aromamischung verzögert von der Matrix freigegeben wird, so dass man eine langanhaltende Wirkung erhält.

Neben üblichen Makrokapseln auf Basis von Gelatine kommen dabei vor allem auch so genannte Mikro- oder Nanokapseln in Betracht. Darunter werden vom Fachmann sphärische Aggregate mit einem Durchmesser im Bereich von etwa 0,0001 bis etwa 5 und vorzugsweise 0,005 bis 0,5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon. Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide). Besonders interessant für die Verkapselung von Zubereitungen für kosmetische Anwendungen sind Koazervate von kationischen Polymeren, insbesondere von Chitosan, mit anioniscchen polymeren, speziell Alginaten. Entsprechende Verfahren sind beispielsweise in den Druckschriften WO 2001 001926, WO 2001 001927, WO 2001 001928 und WO 2001 001929 (Cognis) beschrieben.

Mikrokapseln enthalten die Wirkstoffe häufig in einer Gelphase gelöst oder dispergiert. Als Gelbildner werden vorzugsweise solche Stoffe in Betracht gezogen, welche die Eigenschaft zeigen in wässriger Lösung bei Temperaturen oberhalb von 40 °C Gele zu bilden. Typische Beispiele hierfür sind Heteropolysaccharide und Proteine. Als thermogelierende Heteropolysaccharide kommen vorzugsweise Agarosen in Frage, welche in Form des aus Rotalgen zu gewinnenden Agar-Agar auch zusammen mit bis zu 30 Gew.-% nichtgelbildenden Agaropektinen vorliegen können. Hauptbestandteil der Agarosen sind lineare Polysaccharide aus D-Galaktose und 3,6-Anhydro-L-galaktose, die alternierend β-1,3- und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschiedenen Gelatine-Typen genannt.

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol. Vorzugsweise wird als Verkapselungsmaterial Chitosan eingesetzt. Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

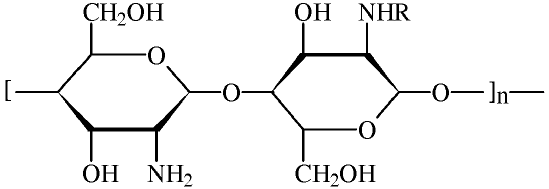

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, wie die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

Die anionischen Polymere haben die Aufgabe, mit den kationischen Membranen zu bilden. Für diesen Zweck eignen sich vorzugsweise Salze der Alginsäure. Bei der Alginsäure handelt es sich um ein Gemisch carboxylgruppenhaltiger Polysaccharide mit folgendem idealisierten Monomerbaustein:

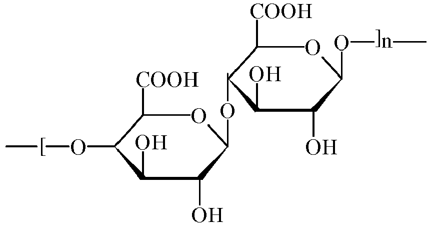

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Alternativ kommen auch Poly(meth)acrylate mit durchschnittlichen Molekulargewichten im Bereich von 5.000 bis 50.000 Dalton sowie die verschiedenen Carboxymethylcellulosen in Frage. Anstelle der anionischen Polymeren können für die Ausbildung der Hüllmembran auch anionische Tenside oder niedermolekulare anorganische Salze, wie beispielsweise Pyrophosphate eingesetzt werden.

Zur Herstellung der Mikrokapseln stellt man üblicherweise eine 1 bis 10, vorzugsweise 2 bis 5 Gew.-%ige wässrige Lösung des Gelbildners, vorzugsweise des Agar-Agars her und erhitzt diese unter Rückfluss. In der Siedehitze, vorzugsweise bei 80 bis 100 °C, wird eine zweite wässrige Lösung zugegeben, welche das Kationpolymer, vorzugsweise das Chitosan in Mengen von 0,1 bis 2, vorzugsweise 0,25 bis 0,5 Gew.-% und den Wirkstoffen in Mengen von 0,1 bis 25 und insbesondere 0,25 bis 10 Gew.-% enthält; diese Mischung wird als Matrix bezeichnet. Die Beladung der Mikrokapseln mit Wirkstoffen kann daher ebenfalls 0,1 bis 25 Gew.-% bezogen auf das Kapselgewicht betragen. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wässrigen oder wässrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben. Nach der Herstellung der Matrix aus Gelbildner, Kationpolymer und Wirkstoffen kann die Matrix optional in einer Ölphase unter starker Scherung sehr fein dispergiert werden, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C zu erwärmen, während man die Ölphase auf 10 bis 20 °C kühlt. Im letzten, nun wieder obligatorischen Schritt erfolgt dann die eigentliche Verkapselung, d.h. die Ausbildung der Hüllmembran durch Inkontaktbringen des Kationpolymers in der Matrix mit den anionischen Polymeren. Hierzu empfiehlt es sich, die gegebenenfalls in der Ölphase dispergierte Matrix bei einer Temperatur im Bereich von 40 bis 100, vorzugsweise 50 bis 60 °C mit einer wässrigen, etwa 1 bis 50 und vorzugsweise 10 bis 15 Gew.-%ige wässrigen Lösung des Anionpolymers zu behandeln und dabei - falls erforderlich - gleichzeitig oder nachträglich die Ölphase zu entfernen. Die dabei resultierenden wässrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise etwa 0,01 bis 1 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig. Alternativ kann man die Anionpolymere auch zur Herstellung der Matrix einsetzen und die Verkapselung mit den Kationpolymeren, speziell den Chitosanen durchführen.

Alternativ kann die Verkapselung auch unter ausschließlicher Verwendung von Kationpolymeren erfolgen, wobei man sich deren Eigenschaft zu Nutze macht, bei pH-Werten oberhalb des pKs-Wertes zu koagulieren.

In einem zweiten alternativen Verfahren wird zur Herstellung der erfindungsgemäßen Mikrokapseln wird zunächst eine O/W-Emulsion zubereitet, welche neben dem Ölkörper, Wasser und den Wirkstoffen eine wirksame Menge Emulgator enthält. Zur Herstellung der Matrix wird diese Zubereitung unter starkem Rühren mit einer entsprechenden Menge einer wässrigen Anionpolymerlösung versetzt. Die Membranbildung erfolgt durch Zugabe der Chitosanlösung. Der gesamte Vorgang findet vorzugsweise im schwach sauren Bereich bei pH = 3 bis 4 statt. Falls erforderlich erfolgt die pH-Einstellung durch Zugabe von Mineralsäure. Nach der Membranbildung wird der pH-Wert auf 5 bis 6 angehoben, beispielsweise durch Zugabe von Triethanolamin oder einer anderen Base. Hierbei kommt es zu einem Anstieg der Viskosität, die durch Zugabe von weiteren Verdickungsmitteln, wie z.B. Polysacchariden, insbesondere Xanthan-Gum, Guar-Guar, Agar-Agar, Alginaten und Tylosen, Carboxymethylcellulose und Hydroxyethylcellulose, höhermolekularen Polyethylenglycolmono- und -diestern von Fettsäuren, Polyacrylaten, Polyacrylamiden und dergleichen noch unterstützt werden kann. Abschließend werden die Mikrokapseln von der wässrigen Phase beispielsweise durch Dekantieren, Filtrieren oder Zentrifugieren abgetrennt.

In einem dritten alternativen Verfahren erfolgt die Bildung der Mikrokapseln um einen vorzugsweise festen, beispielsweise kristallinen Kern, indem dieser schichtweise mit entgegengesetzt geladenen Polyelektrolyten eingehüllt wird. In diesem Zusammenhang sei auf das Europäische Patent EP 1064088 B1 (Max-Planck Gesellschaft) verwiesen.

Zwei weitere Gegenstände der vorliegenden Erfindung betreffen zum einen ein Verfahren zur Maskierung von unangenehmen Geschmackseindrücken bei Zubereitungen zur oralen Aufnahme, bei dem man diesen eine wirksame Menge von mindestens einem der besagten heterozyklischen Neoflavonoid zusetzt und zum anderen die Verwendung er besagten heterozyklischen Neoflavonoide zur Maskierung von unangenehmen Geschmackseindrücken bei Zubereitungen zur oralen Aufnahme.

### Beispiele

### Allgemeine Arbeitsvorschrift (AAV) zur Herstellung der heterozyklischen Neoflavonoide

1,0 Äquivalente des entsprechenden heterocyclischen Zimtsäurederivates werden zu 1,5 Äquivalenten des entsprechenden Phlorogylcinolderivates in 3,0 ml/mmol Dioxan gegeben. Anschließend wird mit 10,0 Äquivalenten konzentrierter Schwefelsäure versetzt und für 7 Stunden zum Rückfluss erhitzt. Nach Abkühlen wird die Reaktionsmischung unter Rühren in zu 40,0 Äquivalenten einer 5 M Natriumbicarbonat Lösung getropft und anschließend nach Zugabe von Essigsäureethylester ausgerührt. Die organische Phase wird noch einmal mit gesättigter Natriumbicarbonatlösung gewaschen, über Natriumsulfat getrocknet und am Rotationsverdampfer eingeengt. Die Aufreinigung kann je nach Problemstellung säulenchromatographisch oder durch Umkristallisation aus Aceton/Wasser erfolgen.

### Beispiel 1

### (4S)-5,7-dihydroxy-4-(4-pyridyl)chroman-2-on (1) und (4R)-5,7-dihydroxy-4-(4-pyridyl)chroman-2-on (2)

Entsprechend der AAV wurden 1,49 g (10,0 mmol) 4-Pyridylacrylsäure mit 1,89 g (15,0 mmol) Phloroglycinol zu einem farblosen Feststoff umgesetzt. Die Ausbeute betrug 1,10 g = 4,3 mmol entsprechend43 % der Theorie)

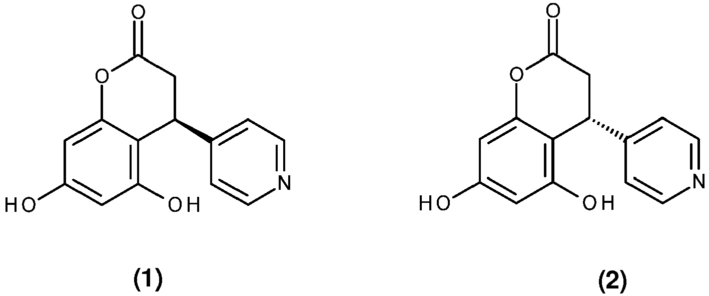

### Beispiel 2

### (4S)-5,7-dihydroxy-4-(3-pyridyl)chroman-2-on (3) und (4R)-5,7-dihydroxy-4-(3-pyridyl)chroman-2-on (4)

Entsprechend der AAV wurden 1,49 g (10,0 mmol) 3-Pyridylacrylsäure mit 1,89 g (15,0 mmol) Phloroglycinol zu einem farblosen Feststoff umgesetzt. Die Ausbeute betrug 32 g = 1,2 mmol, entsprechend 12 % der Theorie)

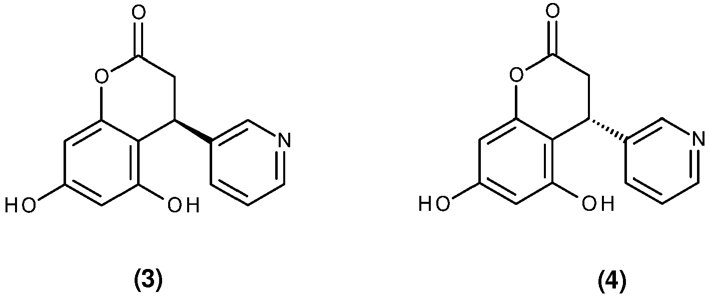

### Anwendungsbeispiel 1

### Bitter-Reduzierung einer Bitterstofflösung

Um die Reduzierung (d.h. die Maskierung oder Verminderung) des Bitter-Eindrucks in einer Probe zu quantifizieren, wurde die Bitterkeit einer Lösung enthaltend 500 ppm Coffein oder 300 ppm Theobromin oder 100 ppm Naringin von einer Expertengruppe jeweils mit einer Probe verglichen, die 500 ppm Coffein oder 300 ppm Theobromin oder 100 ppm Naringin und zusätzlich die jeweils angegebene Menge einer (hinsichtlich der Fähigkeit zur Bitter-Reduzierung) zu beurteilenden Substanz enthielt (Einstufung: 1 [nicht bitter] bis 10 [extrem bitter]). Für die Auswertung, d.h. die Berechnung der Reduktion (in %) des Bittereindrucks wurden jeweils die Durchschnittswerte der Einschätzungen der Expertengruppe verwendet. Die Ergebnisse sind in **Tabelle 1** zusammengefasst:

**Tabelle 1**

| Bitter-reduzierung einer Bitterstofflösung | | | | |
|---|---|---|---|---|
| **Test-Substanz** | **Bitterstoff** | **Bitter-Eindruck (1-10)** | | **% Reduktion des Bitter-Eindrucks (Signifikanz)** |
| | | ohne Test-substanz | mit Testsubstanz | |
| 25 ppm **(1)** + **(2)** (Verhältnis 1:1) | 100 ppm Naringin | 6,6 ± 2,0 | 3,2 + 1,5 | 52,1 % (p < 0,0001) |
| 10 ppm **(1)** + **(2)** (Verhältnis 1:1) | 500 ppm Coffein | 3,8 ± 1,3 | 2,4 + 1,0 | 37,0% (p < 0,0007) |
| 25 ppm **(1)** + **(2)** (Verhältnis 1:1) | 300 ppm Theobromin | 3,6 ± 2,0 | 2,9 ± 1,4 | 19,4 % (p < 0,25) |
| 50 ppm **(3)** + **(4)** (Verhältnis 1:1) | 100 ppm Naringin | 5,0 ± 2,0 | 3,3 ± 1,8 | 34,1 % (p < 0,02) |
| 50 ppm **(3)** + **(4)** (Verhältnis 1:1) | 500 ppm Coffein | 3,5 ± 1,4 | 3,1 ± 1,7 | 13,7 % (p < 0,35) |
| 25 ppm **(3)** + **(4)** (Verhältnis 1:1) | 300 ppm Theobromin | 4,2 ±2,2 | 3,4 ± 1,8 | 18,8 % (p < 0,25) |

### Anwendungsbeispiel 2

In der folgenden **Tabelle 2** sind verschiedene Formulierungen zur Maskierung eines Bittergeschmackes wiedergegeben.

**Tabelle 2**

| Anwendungsformulierungen | | | | | | | | |
|---|---|---|---|---|---|---|---|---|
| **Inhaltsstoff** | **Mischung (Einsatz in Gew.-%)** | | | | | | | |
| | **A** | **B** | **C** | **D** | **E** | **F** | **G** | **H** |
| **(1)** + **(2)** (Verhältnis 1:1) | 5 | 7 | 10 | 15 | 15 | 20 | 20 | 25 |
| Homoeriodictyol | 5 | - | 2,5 | - | - | - | - | - |
| Eriodictyol | - | 2,5 | - | - | - | - | - | - |
| Phloretin | - | - | - | 1 | - | - | - | - |
| Hesperetin | - | - | - | - | 0,5 | - | - | - |
| 10 Gew.-% trans-Pellitorin (z.B. nach WO 2004/043906) in 1,2-Propylenglycol/ Diethylmalonat | - | - | - | 0,25 | 0,25 | 0,5 | 0,25 | - |
| Extrakt aus *Hydrangea macrophylla* gemäß EP 2 298 084 A1, enthaltend Phyllodulcin | 2,5 | - | 2,5 | - | - | - | - | 2,5 |
| Extrakt aus *Rubus suavissimus* enthaltend Rubusosid gemäß der europäischen Patentanmeldung mit der Anmeldenummer 11 165 566.8 (Symrise) | - | 2,5 | 2,5 | - | - | - | - | - |
| 1,2-Propylenglycol | - | 20 | - | ad 100 | ad 100 | 20 | ad 100 | - |
| Glycerin | - | ad 100 | - | 20 | 20 | ad 100 | 20 | - |
| Maltodextrin | ad 100 | - | ad 100 | - | - | - | - | ad 100 |

Die Stoffe bzw. Lösungen werden in den oben angegebenen Mengenverhältnissen gemischt und dann mit 1,2-Propylenglycol und/oder Glycerin aufgenommen und durch leichtes Erwärmen vollständig gelöst bzw. mit den festen Trägerstoffen homogen gemischt.

### Anwendungsbeispiel 3

In der folgenden **Tabelle 3** sind Beispiele für sprühgetrocknete Halbfertigware angegeben. Zur Herstellung wurde das Trinkwasser in einem Behälter vorgelegt und das Maltodextrin und das Gummi Arabicum darin gelöst. Anschließend wurde Verbindung **(1) + (2)** und/oder Verbindung **(3) + (4)** mit einem Turrax in die Trägerstofflösung emulgiert. Die Temperatur der Sprühlösung sollte dabei 30°C nicht überschreiten. Das Gemisch wurde dann sprühgetrocknet (Solltemperatur Eingang: 185 - 195°C, Solltemperatur Ausgang: 70 - 75°C). Die resultierende sprühgetrocknete Halbfertigware enthielt ca. 18 - 22 % der Verbindungen **(1) + (2)** und/oder **(3) + (4).**

**Tabelle 3**

| Anwendungsformulierungen | | | |
|---|---|---|---|
| **Inhaltsstoff** | **Einsatz in Gew.-%** | | |
| | **A** | **B** | **C** |
| Trinkwasser | 60,8 | 60,8 | 60,8 |
| Maltodextrin aus Weizen | 24,3 | 24,3 | 24,3 |
| Gummi Arabicum | 6,1 | 6,1 | 6,1 |
| **(1)** + **(2)** (Verhältnis 1:1) | 8,8 | --- | 4,4 |
| **(3)** + **(4)** (Verhältnis 1:1) | --- | 8,8 | 4,4 |

### Anwendungsbeispiel 4

In der folgenden **Tabelle 4** sind verschiedene Tee-Zubereitungen angegeben. Zu ihrer Herstellung wurden der Tee und die Halbfertigware gemischt und in Teebeutel aus Filterpapier abgepackt (je 2 g pro Filterbeutel). Zur Anwendung wird ein Teebeutel in 100 - 250 ml kochendes Wasser aufgegossen und 2 - 5 min ziehen gelassen.

**Tabelle 4**

| Anwendungsformulierungen | | | |
|---|---|---|---|
| **Inhaltsstoff** | **Einsatz in Gew.-%** | | |
| | **A** | **B** | **C** |
| Schwarzer Tee, Ceylon, Blattware | 94 | - | - |
| Grüner Tee, China, Blattware | - | 92 | - |
| Mate Tee, Peru, Blattware | - | - | 95 |
| Mischung A aus Anwendungsbeispiel 2 | 6 | - | - |
| Mischung B aus Anwendungsbeispiel 2 | - | 8 | - |
| Mischung C aus Anwendungsbeispiel 2 | - | - | 5 |

### Anwendungsbeispiel 5

In der folgenden **Tabelle 5** ist eine Schwarztee-Zubereitung angegeben. Zu ihrer Herstellung wurden der Tee und die Halbfertigwaren gemischt und in Teebeutel aus Filterpapier abgepackt (je 2 g pro Filterbeutel). Zur Anwendung wird ein Teebeutel in 100 - 250 ml kochendes Wasser aufgegossen und 2 - 5 min ziehen gelassen.

**Tabelle 5**

| Anwendungsformulierungen | |
|---|---|
| **Inhaltsstoff** | **Einsatz in Gew.-%** |
| Schwarzer Tee, Ceylon, Blattware | 94 |
| Mischung A aus Anwendungsbeispiel 2 | 3 |
| Mischung B aus Anwendungsbeispiel 2 | 3 |

### Anwendungsbeispiel 6

In der folgenden **Tabelle 6** sind zwei Formulierungen für ein Eistee-Getränk (Schwarztee) angegeben. Die Verbindungen (1) und (2), bzw. (3) und (4) wurden jeweils 10 % in Ethanol vorgelöst. Schwarztee-Extrakt wurde in Wasser gelöst und zusammen mit Zucker, einer Aromazubereitung (Pfirsichgeschmack), sowie den ethanolischen Lösungen der Verbindungen (1) und (2) (Zubereitung A), bzw. (3) und (4) (Zubereitung B) in einem Becherglas verrührt.

**Tabelle 6**

| Anwendungsformulierungen | | |
|---|---|---|
| **Inhaltsstoff** | **Einsatz in Gew.-%** | |
| | **A** | **B** |
| Schwarztee-Extrakt | 1,4 | 1,4 |
| Wasser | 89,5 | 89,5 |
| Aromazubereitung (Typ Pfirsich) | 0,67 | 0,67 |
| Zucker | 7 | 7 |
| Citronensäure (kristallin) | 1,2 | 1,2 |
| Ascorbinsäure | 0,2 | 0,2 |
| **(1)** und **(2)** im Verhältnis 1:1 als 10%ige Lösung in Ethanol | 0,03 | - |
| **(3)** und **(4)** im Verhältnis 1:1 als 10%ige Lösung in Ethanol | - | 0,03 |

### Anwendungsbeispiel 7

In der folgenden **Tabelle 7** sind zwei weitere Formulierungen für ein Eistee-Getränk (Grüntee, zuckerreduziert) angegeben. Die Verbindungen (1) und (2) bzw. (3) und (4) wurden jeweils 10 % in Ethanol vorgelöst. Grüntee-Extrakt wurde in Wasser gelöst und zusammen mit Zucker, sowie dem Süßstoff Saccharin bzw. Rebaudiosid A, einer Aromazubereitung (Zitronengeschmack), sowie den ethanolischen Lösungen der Verbindungen (1) und (2) bzw. (3) und (4) (Zubereitung B) in einem Becherglas verrührt.

**Tabelle 7**

| Anwendungsformulierungen | | |
|---|---|---|
| **Inhaltsstoff** | **Einsatz in Gew**.-**%** | |
| | **A** | **B** |
| Grüntee-Extrakt | 1,4 | 1,4 |
| Wasser | 92,95 | 93,03 |
| Aromazubereitung (Typ Zitrone) | 0,65 | 0,65 |
| Zucker | 3,45 | 3,45 |
| Süßstoff Saccharin | 0,1 | --- |
| Süßstoff Rebaudiosid A | --- | 0,02 |
| Citronensäure (kristallin) | 1,2 | 1,2 |
| Ascorbinsäure | 0,2 | 0,2 |
| **(1)** und **(2)** im Verhältnis 1:1, 10%ige Lösung in Ethanol | 0,05 | --- |
| **(3)** und **(4)** im Verhältnis 1:1, 10%ige Lösung in Ethanol | --- | 0,05 |

### Anwendungsbeispiel 8

In der folgenden **Tabelle 8** sind zwei weitere Formulierungen für ein Eistee-Getränk (Schwarztee, zuckerfrei) angegeben. Die Verbindungen (1) und (2) bzw. (3) und (4) wurden jeweils in Ethanol vorgelöst. Schwarztee-Extrakt wurde in Wasser gelöst und zusammen mit dem Süßstoff Saccharin, einer Aromazubereitung (Zitronengeschmack), sowie den ethanolischen Lösungen der Verbindungen (1) und (2) (Zubereitung A), bzw. (3) und (4) (Zubereitung B) in einem Becherglas verrührt.

**Tabelle 8**

| Anwendungsformulierungen | | |
|---|---|---|
| **Inhaltsstoff** | **Einsatz in Gew.-%** | |
| | **A** | **B** |
| Schwarztee-Extrakt | 1,4 | 1,4 |
| Wasser | 96,465 | 96,465 |
| Saccharin | 0,035 | 0,035 |
| Aromazubereitung (Typ Zitrone) | 0,65 | 0,65 |
| Citronensäure (kristallin) | 1,2 | 1,2 |
| Ascorbinsäure | 0,2 | 0,2 |
| **(1)** und **(2)** im Verhältnis 1:1, 10%ige Lösung in Ethanol | 0,05 | --- |
| **(3)** und **(4)** im Verhältnis 1:1, 10%ige Lösung in Ethanol | --- | 0,05 |

### Anwendungsbeispiel 9

In der folgenden **Tabelle 9** sind Formulierungen für ein lösliches Cappuccino-Getränk angegeben. Die angegebenen Rohstoffe wurden vermischt. Jeweils 12,5 g des hergestellten Instant-Cappuccino-Pulvers wurden in 150 ml heißem Wasser gelöst.

**Tabelle 9**

| Anwendungsformulierungen | | | |
|---|---|---|---|
| **Inhaltsstoff** | **Einsatz in Gew.-%** | | |
| | **A** | **B** | **C** |
| Kaffee-Extrakt, sprühgetrocknet | 18,0 | 18,0 | 16,0 |
| Zucker | 26,3 | 26,3 | 30,8 |
| Fettpulver | 18,2 | 18,2 | 18,2 |
| Kaffeeweißer, schäumend | 30,0 | 30,0 | 28,0 |
| Hydrokolloide/ Emulgatoren | 1,8 | 1,8 | 1,8 |
| Lactose | 4,7 | 4,7 | 4,7 |
| Mischung A aus Anwendungsbeispiel 2 | 1,0 | - | - |
| Mischung C aus Anwendungsbeispiel 2 | - | 1,0 | - |
| Halbfertigware C aus Anwendungsbeispiel 3 | - | - | 0,5 |

### Anwendungsbeispiel 10

In der folgenden **Tabelle 10** sind Formulierungen für ein Soja-Getränk angegeben. Die Verbindungen (1) und (2) bzw. (3) und (4) wurden jeweils in Ethanol vorgelöst und zu einer Sojamilch aus einem lokalen Supermarkt hinzugefügt. Die Mischung wurde zusammen mit dem Milcharoma im Becherglas verrührt.

**Tabelle 10**

| Anwendungsformulierungen | | | | |
|---|---|---|---|---|
| **Inhaltsstoff** | **Einsatz in Gew.-%** | | | |
| | **A** | **B** | **C** | **D** |
| Sojamilch (nicht aromatisiert, ungesüßt) | 96,775 | 99,75 | 98,33 | 97,65 |
| Vanillearoma | 0,1 | 0,1 | - | 0,05 |
| Milcharoma | - | - | 0,1 | 0,05 |
| Saccharose | 3,0 | - | 1,5 | 2,0 |
| Sucralose | - | 0,025 | 0,01 | - |
| Na-Saccharin | - | - | 0,01 | - |
| Emulgum | 0,1 | 0,1 | - | 0,1 |
| **(1)** und **(2)** im Verhältnis 1:1, 10%ige Lsg. in Ethanol | 0,025 | - | 0,05 | - |
| **(3)** und **(4)** im Verhältnis 1:1, 10%ige Lsg. in Ethanol | - | 0,025 | - | 0,05 |
| Hesperetin, 5 %ig in Ethanol | - | - | - | 0,1 |

### Anwendungsbeispiel 11

In der folgenden **Tabelle 11** ist eine Formulierung für ein Soja-Getränk in Kombination mit γ-Aminobuttersäure angegeben. Die γ-Aminobuttersäure wurde in Wasser und die Verbindungen (1) und (2) in Ethanol vorgelöst und zu einer Sojamilch aus einem lokalen Supermarkt hinzugefügt. Die resultierende Mischung wurde zusammen mit dem Milcharoma im Becherglas verrührt.

**Tabelle 11**

| Anwendungsformulierungen | |
|---|---|
| **Inhaltsstoff** | **Einsatz in Gew.-%** |
| Sojamilch (aus lokalem Supermarkt) | 99,75 |
| Milcharoma | 0,1 |
| **(1)** und **(2)** im Verhältnis 1:1, 10%ige Lösung in Ethanol | 0,05 |
| γ-Aminobuttersäure, 1%ig in Wasser | 0,1 |

### Anwendungsbeispiel 12

In der folgenden **Tabelle 12** ist eine Formulierung für einen Grapefruitsaft angegeben. Die Verbindungen (1) und (2) wurden in Ethanol vorgelöst und zu einem Grapefruitsaft aus einem lokalen Supermarkt hinzugefügt. Die resultierende Mischung wurde in einem Becherglas durch Rühren homogenisiert.

**Tabelle 12**

| Anwendungsformulierungen | |
|---|---|
| **Inhaltsstoff** | **Einsatz in Gew.-%** |
| Grapefruitsaft (aus lokalem Supermarkt) | 99,975 |
| **(1)** und **(2)** im Verhältnis 1:1, 10%ig in Ethanol | 0,025 |

### Anwendungsbeispiel 13

In der folgenden **Tabelle 13** sind zwei Formulierungen für bittere Schokolade angegeben. Dazu wurde eine bittere Schokolade aus folgenden Rohstoffen hergestellt und anschließend in rechteckigen Formen ausgegossen:

**Tabelle 13**

| Anwendungsformulierungen | | |
|---|---|---|
| **Inhaltsstoff** | **Einsatz in Gew.-%** | |
| | **A** | **B** |
| Kakaomasse | Ad 100 | Ad 100 |
| Kakaobutter | 11,70 | 11,70 |
| Zucker | 29,50 | 29,50 |
| Magermilch | 3,00 | 3,00 |
| Lecithin | 0,2 | 0,2 |
| Vanillin | 0,035 | 0,035 |
| **(1)** und **(2)** im Verhältnis 1:1, 10%ig in Ethanol | - | 0,025 |
| **(3)** und **(4)** im Verhältnis 1:1, 10%ig in Ethanol | 0,05 | - |

### Anwendungsbeispiel 14

In der folgenden **Tabelle 14** ist eine Formulierung für ein Kaugummi wiedergegeben. Die Teile A bis D wurden gemischt und intensiv geknetet. Die Rohmasse kann z.B. in Form von dünnen Streifen zu verzehrfertigen Kaugummis verarbeitet werden.

**Tabelle 14**

| Anwendungsformulierungen | | |
|---|---|---|
| **Teil** | **Inhaltsstoff** | **Einsatz in Gew.-%** |
| A | Kaugummibase, Company "Jagum T" | 30,00 |
| B | Sorbit, pulverisiert | 39,00 |
| | Isomalt^{®} (Palatinit GmbH) | 9,50 |
| | Xylit | 2,00 |
| | Mannit | 3,00 |
| | Aspartam^{®} | 0,10 |
| | Acesulfam^{®} K | 0,10 |
| | Emulgum^{®} (Colloides Naturels, Inc.) | 0,30 |
| C | Sorbitol, 70%ig in Wasser | 14,00 |
| | Glycerin | 1,00 |
| D | Aroma, enthaltend 1 % der Verbindungen **(1)** und **(2)** im Verhältnis 1:1, bezogen auf das Gesamtgewicht des Aromas | 1,00 |

### Anwendungsbeispiel 15

In der folgenden **Tabelle 15** ist eine Formulierung für ein Kaugummi wiedergegeben. Die Inhaltsstoffe der Teile A und B wurden jeweils für sich vorgemischt und zusammen unter Vakuum bei 25 - 30°C 30 min gut verrührt. Teil C wurde vorgemischt und zu A und B gegeben; D wurde hinzugefügt und die Mischung unter Vakuum bei 25 - 30°C für 30 min gut verrührt. Nach Entspannung war die Zahnpasta fertig und konnte abgefüllt werden.

**Tabelle 15**

| Anwendungsformulierungen | | |
|---|---|---|
| **Teil** | **Inhaltsstoff** | **Einsatz in Gew.-%** |
| | demineralisiertes Wasser | 21,50 |
| A | Sorbitol (70%) | 45,00 |
| | Solbrol^{®} M, Natriumsalz (Bayer AG, p-Hydroxybenzoesäurealkylester) | 0,15 |
| | Trinatriumphosphat | 0,10 |
| | Saccharin, 450 fach | 0,20 |
| | Natriummonofluorphosphat | 1,12 |
| | Polyethylenglycol 1500 | 5,00 |
| B | Sident 9 (abrasives Siliciumdioxid) | 10,00 |
| | Sident 22 S (verdickendes Siliciumdioxid) | 8,00 |
| | Natriumcarboxymethylcellulose | 0,90 |
| | Titandioxid | 0,50 |
| C | demineralisiertes Wasser | 4,53 |
| | Natriumlaurylsulfat | 1,50 |
| D | Aroma, enthaltend 1 Gew.-% der Verbindungen **(1)** und **(2)** im Verhältnis 1:1, bezogen auf das Gesamtgewicht des Aromas | 1,50 |

## Patentansprüche

1. Heterozyklische Neoflavonoide der Formel (II) 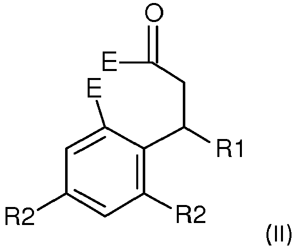 in der
E jeweils OH oder beide E zusammen Sauerstoff bedeuten,
R¹ einen 6-gliedrigen heteroaromatischen Rest darstellt, der ausgewählt ist aus der Gruppe bestehend aus, 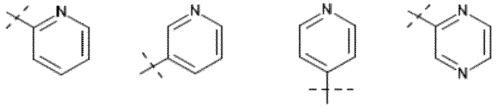
R² jeweils Wasserstoff, Hydroxy, Ethoxy, n-Propoxy oder iso-Propoxy bedeutet,
und deren physiologisch verträgliche Salze.

2. Heterozyklische Neoflavonoide der Formeln (1) bis (6) nach Anspruch 1 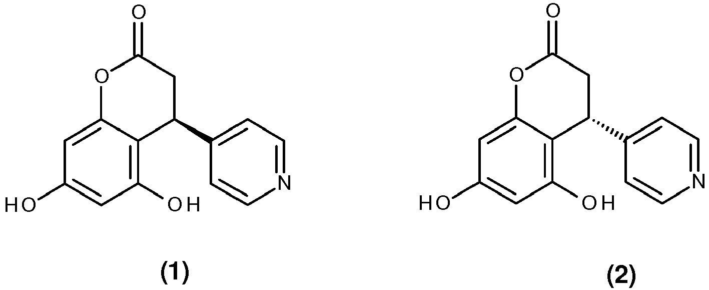 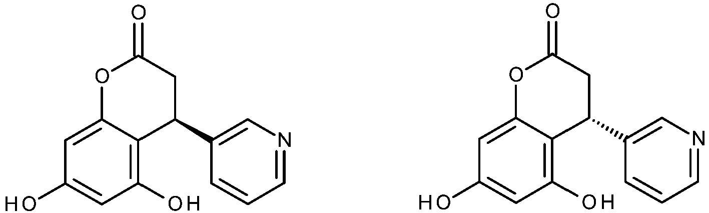 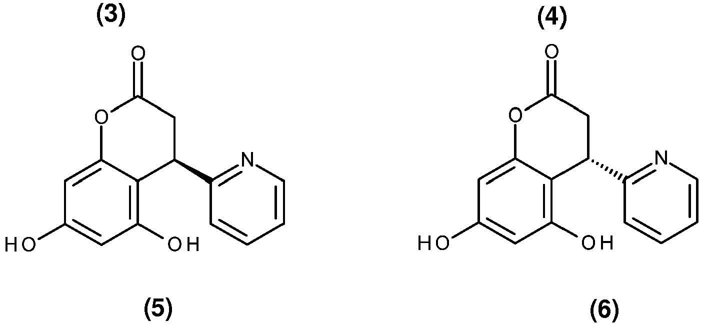 und deren physiologisch verträglichen Salze.

3. Heterozyklische Neoflavonoide nach mindestens einem der Ansprüche 1 bis 2, **dadurch gekennzeichnet, dass** die Kationen der physiologisch verträglichen Salze ausgewählt sind aus der Gruppe, die gebildet wird von Na⁺, K⁺, NH₄⁺, Ca²⁺, Mg²⁺, Al³⁺ und Zn²⁺ sowie deren Gemischen.

4. Zubereitungen zur oralen Aufnahme, enthaltend
(a) mindestens ein heterozyklisches Neoflavonoid nach den Ansprüchen 1 und/oder 2, und
(b) mindestens einen Stoff mit bitterem, adstringierendem und/oder metallischem Geschmackseindruck.

5. Zubereitungen nach Anspruch 4, **dadurch gekennzeichnet, dass** sie als Komponente (b) Stoffe enthalten, die ausgewählt sind aus der Gruppe, die gebildet wird von Xanthinalkaloiden, Xanthinen, Alkaloiden, phenolischen Glycosiden, Flavanoidglycosiden, Chalconen oder Chalconglycosiden, Dihydrochalconglycosiden , hydrolisierbaren Tanninen, nichthydrolisierbaren Tanninen, Flavonen und deren Glycosiden, andere Polyphenolen, terpenoiden Bitterstoffen, Absinthin aus Wermut, Amarogentin aus Enzian, metallischen Salzen, Vitaminen, Denatoniumsalzen, Sucraloseoctaacetat, Harnstoff, ungesättigte Fettsäuren, Aminosäuren, Peptide und deren Gemischen.

6. Zubereitungen nach Anspruch 4, **dadurch gekennzeichnet, dass** sie als Komponente (b) Stoffe enthalten, die ausgewählt sind aus der Gruppe, die gebildet wird von Catechinen und Proanthocyanidinen, Coffein und Theobromin, Naringin und Süßstoffen.

7. Zubereitungen nach mindestens einem der Ansprüche 4 bis 6, **dadurch gekennzeichnet, dass** es sich dabei um Nahrungsmittel, Mund- und Zahnreinigungs- und -pflegemittel oder pharmazeutische Zubereitungen handelt.

8. Zubereitungen nach mindestens einem der Ansprüche 4 bis 7, **dadurch gekennzeichnet, dass** es sich um Backwaren, Süßwaren, alkoholische oder nicht- alkoholische Getränke, Erfrischungsgetränke, Nektare, Schorlen, Obst- und Gemüsesäfte, Frucht- oder Gemüsesaftzubereitungen, Instantgetränke, Fleischprodukte, Eier oder Eiprodukte, Getreideprodukte, Milchprodukte, Produkte aus Sojaprotein oder anderen Sojabohnen-Fraktionen, Produkte aus anderen pflanzlichen Proteinquellen, Fruchtzubereitungen, Gemüsezubereitungen, Knabberartikel, Produkte auf Fett- und Ölbasis oder Emulsionen derselben, sonstige Fertiggerichte und Suppen, Gewürze, Würzmischungen sowie insbesondere Aufstreuwürzungen, Zahnpasten, Mundwässer, Erkältungsmittel oder Wirkstoffkapseln handelt.

9. Zubereitungen nach mindestens einem der Ansprüche 4 bis 8, **dadurch gekennzeichnet, dass** die Gesamtmenge der Komponenten (a) und (b) bezogen auf die konfektionierten Endprodukte 0,001 bis 1 Gew.-% beträgt.

10. Verfahren zur Maskierung von unangenehmen Geschmackseindrücken bei Zubereitungen zur oralen Aufnahme, bei dem man diesen eine wirksame Menge von mindestens einem heterozyklischen Neoflavonoid nach den Ansprüchen 1 und/oder 2 zusetzt.

11. Verwendung von heterozyklischen Neoflavonoiden nach den Ansprüchen 1 und/ oder 2 zur Maskierung von unangenehmen Geschmackseindrücken bei Zubereitungen zur oralen Aufnahme.

12. Verwendung nach Anspruch 11, **dadurch gekennzeichnet, dass** man die heterozyklischen Neoflavonoide in Mengen von 0,1 mg/kg bis 1 g/kg - berechnet auf die Halbfertigwaren oder die konfektionierten Endprodukte einsetzt.

## Claims

1. Heterocyclic neoflavonoids of formula (II) 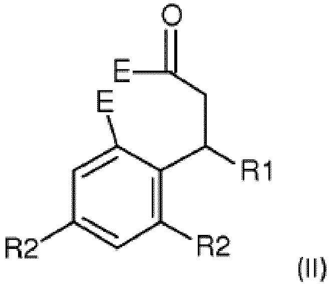 in which
E is independently of another OH or both E together are oxygen,
R1 is a 6-memberes heteroaromatic rest, which is selected from the group consistng of 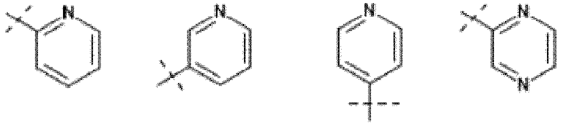
R2 is independently of another hydrogen, hydroxy, ethoxy, n-propoxy or iso-propoxy, and their physiological accepted salts.

2. Heterocyclic neoflavonoids of formula (1) to (6) according to claim 1 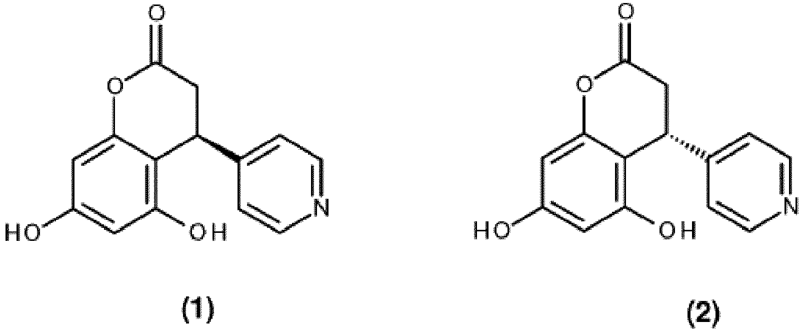 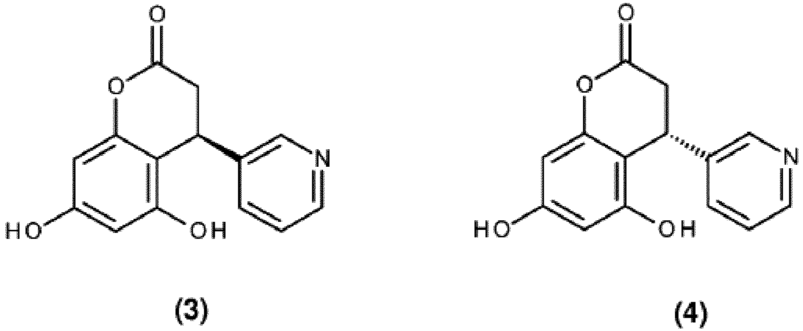 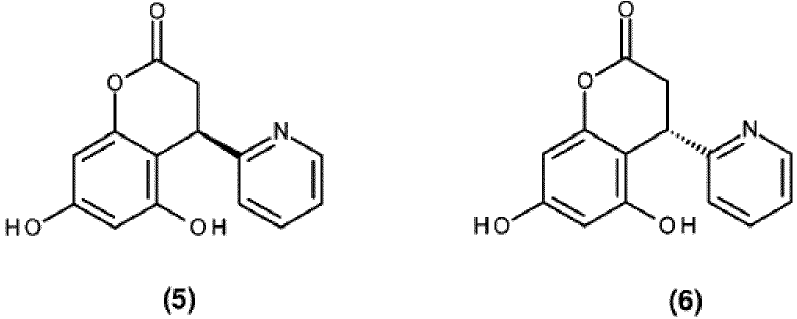 and their physiological accepted salts.

3. Heterocyclic neoflavonoids according to at least one of the claims 1 to 2, **characterized in that** the cations of the physiological accepted salts are selected from the group consisting of Na⁺, K⁺, NH₄⁺, Ca²⁺, Mg²⁺, Al³⁺ and Zn²⁺ as well as their mixtures.

4. Oral preparations, including
(a) at least one heterocyclic neoflavonoid according to claims 1 and/or 2, and
(b) at least one compound with a bitter, astringent and/or metallic taste impression.

5. Oral preparations according to claim 4, **characterized in that** it contains compounds as component (b) which are selected from the group consisting of xanthine alkaloids, xanthines, alkaloids, phenolic glycosides, flavanoidoglycosides, chalcones or chalconglyco-sides, dihydrochalconglycosides, hydrolyzable tannins, non-hydrolizable tannins, flavones and their glycosides, other polyphenols, terpenoid bittering agents, absinthin from vermouth, aminogentin from gentian, metallic salts, vitamins, denatonium salts, sucralose octaacetate, urea, unsaturated fatty acids, amino acids, peptides and mixtures thereof.

6. Oral preparations according to claim 4, **characterized in that** it contains compounds as component (b) which are selected from the group consisting of catechins and proanthocyanidines, caffeine and theobromine, naringine and sweeteners.

7. Oral preparations according to at least one of the claims 4 to 6, **characterized in that** they are foodstuffs, oral and dental cleansing agents or pharmaceutical preparations.

8. Oral preparations according to at least one of the claims 4 to 7, **characterized in that** they are bakery products, confectionery, alcoholic or non-alcoholic beverages, soft drinks, nectars, spritzer, fruit and vegetable juices, fruit or vegetable juice preparations, instant beverages, meat products, eggs or egg products, cereal products, dairy products, products of soy protein or other soybean fractions, products from other plant protein sources, fruit preparations, vegetable preparations, snack products, fat and oil based products or emulsions thereof, other ready-to-serve meals and soups, seasonings, spice mixes and in particular spices to sprinkle, toothpastes, mouthwashes, cold products or active ingredient capsules.

9. Oral preparations according to at least one of the claims 4 to 8, **characterized in that** the total amount of the components (a) and (b) is from 0.001 to 1% by weight, based on the finished products.

10. A method for masking unpleasant taste impressions in oral preparations comprising adding thereto an effective amount of at least one heterocyclic neoflavonoid according to claims 1 and/or 2.

11. Use of heterocyclic neoflavonoids according to claims 1 and/or 2 for masking unpleasant taste impressions in oral preparations

12. Use according to claim 11, **characterized in that** the heterocyclic neoflavonoids are used in amounts of from 0.1 mg/kg to 1 g/kg - calculated on the semi-finished products or the finished products.

## Revendications

1. Néoflavonoïdes hétérocycliques de formule (II) 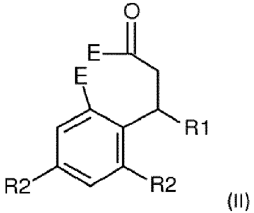 dans laquelle
les E signifient chacun OH ou les deux E signifient ensemble oxygène,
R1 représente un radical hétéroaromatique à 6 éléments, qui est choisi dans le groupe constitué par : 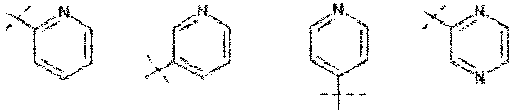
R2 signifie à chaque fois hydrogène, hydroxy, éthoxy, n-propoxy ou iso-propoxy,
et leurs sels physiologiquement compatibles.

2. Néoflavonoïdes hétérocycliques des formules (1) à (6) selon la revendication 1 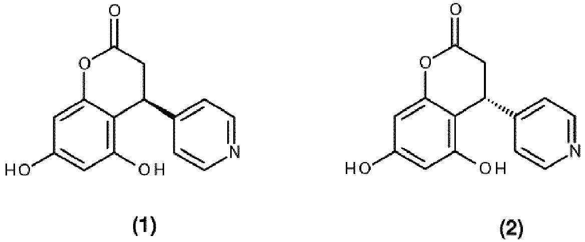 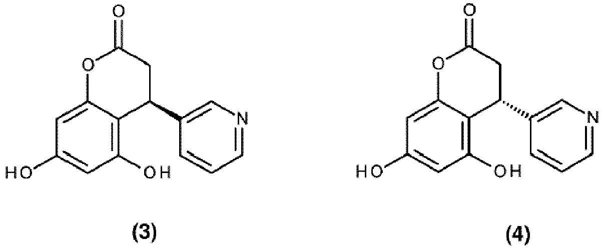 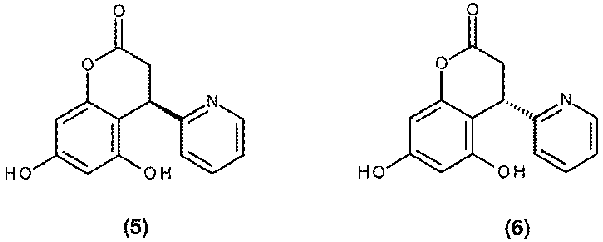 et leurs sels physiologiquement compatibles.

3. Néoflavonoïdes hétérocycliques selon au moins l'une quelconque des revendications 1 à 2, **caractérisés en ce que** les cations des sels physiologiquement compatibles sont choisis dans le groupe formé par Na+, K+, NH4+, Ca2+, Mg2+, Al3+ et Zn2+, ainsi que leurs mélanges.

4. Préparations pour ingestion orale, contenant :
(a) au moins un néoflavonoïde hétérocyclique selon les revendications 1 et/ou 2, et
(b) au moins une substance à goût amer, astringent et/ou métallique.

5. Préparations selon la revendication 4, **caractérisées en ce qu'**elles contiennent en tant que composant (b) des substances choisies dans le groupe formé par les alcaloïdes de xanthine, les xanthines, les alcaloïdes, les glycosides phénoliques, les glycosides de flavonoïdes, les chalcones ou les glycosides de chalcones, les glycosides de dihydrochalcone, les tannins hydrolysables, les tannins non hydrolysables, les flavones et leurs glycosides, les autres polyphénols, les substances amères terpénoïdes, l'absinthe d'armoise, l'amarogentine de gentiane, les sels métalliques, les vitamines, les sels de dénatonium, l'octaacétate de sucralose, l'urée, les acides gras insaturés, les acides aminés, les peptides et leurs mélanges.

6. Préparations selon la revendication 4, **caractérisées en ce qu'**elles contiennent en tant que composant (b) des substances choisies dans le groupe formé par les catéchines et les proanthocyanidines, la caféine et la théobromine, la naringine et les édulcorants.

7. Préparations selon au moins l'une quelconque des revendications 4 à 6, **caractérisées en ce qu'**il s'agit de produits alimentaires, de produits de nettoyage et de soin de la bouche et des dents ou de préparations pharmaceutiques.

8. Préparations selon au moins l'une quelconque des revendications 4 à 7, **caractérisées en ce qu'**il s'agit de produits de boulangerie, de confiseries, de boissons alcooliques ou non alcooliques, de boissons rafraîchissantes, de nectars, de limonades, de jus de fruits et de légumes, de préparations à base de jus de fruits ou de légumes, de boissons instantanées, de produits à base de viande, d'oeufs ou de produits à base d'oeufs, de produits céréaliers, de produits laitiers, de produits à base de protéine de soja ou d'autres fractions de soja, de produits d'autres sources de protéines végétales, de préparations à base de fruits, de préparations à base de légumes, d'en-cas, de produits à base de graisse et d'huile ou leurs émulsions, d'autres plats préparés et potages, d'épices, de mélanges d'épices, ainsi que notamment d'assaisonnements à saupoudrer, de dentifrices, de bains de bouche, de remèdes contre les refroidissements ou de gélules d'agents actifs.

9. Préparations selon au moins l'une quelconque des revendications 4 à 8, **caractérisées en ce que** la quantité totale des composants (a) à (b) par rapport aux produits finaux conditionnés est de 0,001 à 1 % en poids.

10. Procédé de masquage de goûts désagréables dans des préparations pour ingestion orale, selon lequel une quantité efficace d'au moins un néoflavonoïde hétérocyclique selon les revendications 1 et/ou 2 est ajoutée à celles-ci.

11. Utilisation de néoflavonoïdes hétérocycliques selon les revendications 1 et/ou 2 pour le masquage de goûts désagréables dans des préparations pour ingestion orale.

12. Utilisation selon la revendication 11, **caractérisée en ce que** les néoflavonoïdes hétérocycliques sont utilisés en quantités de 0,1 mg/kg à 1 g/kg, calculées par rapport aux produits semi-finis ou aux produits finaux conditionnés.
